# EUROPÄISCHE PATENTSCHRIFT

(11) **EP 0 563 358 B1**
(45) Veröffentlichungstag und Bekanntmachung des Hinweises auf die Patenterteilung: **20.09.1995**
(21) Anmeldenummer: 92922093.7
(22) Anmeldetag: 09.10.1992
(51) Int. Cl.: C07C 405/00, A61K 31/557

(54) **9-SUBSTITUIERTE BICYCLO [3.3.0]OCTAN-DERIVATE VERWENDBAR ALS TXA2-ANTAGONISTEN**
9-SUBSTITUTED BICYCLO [3.3.0]OCTANE DERIVATIVES, FOR USE AS TXA2 ANTAGONISTS
DERIVES BICYCLO [3.3.0]OCTANES 9-SUBSTITUES, POUVANT ETRE UTILISES COMME ANTAGONISTES DE TXA2

(30) Priorität: 11.10.1991 DE 4134156
(43) Veröffentlichungstag der Anmeldung: 06.10.1993
(73) Patentinhaber: SCHERING AKTIENGESELLSCHAFT, 13353 Berlin (DE)
(72) Erfinder: KLAR, Ulrich, D-1000 Berlin 27 (DE); VORBRÜGGEN, Helmut, D-1000 Berlin 27 (DE); REHWINKEL, Hartmut, D-1000 Berlin 44 (DE); THIERAUCH, Karl-Heinz, D-1000 Berlin 37 (DE); VERHALLEN, Peter, D-1000 Berlin 28 (DE)
(86) Internationale Anmeldenummer: DE9200865
(87) Internationale Veröffentlichungsnummer: WO9307118

(56) Entgegenhaltungen:
- EP-A- 0 086 611
- EP-A- 0 224 275
- WO-A-89/00990
- DE-A- 3 428 266

## Beschreibung

Die Erfindung betrifft 9-substituierte Bicyclo[3.3.0]octan-Derivate, Verfahren zu ihrer Herstellung sowie ihre Verwendung als Hilfsstoffe für pharmakologische Untersuchungen und als Arzneimittel.
Bicyclo[3.3.0]octan-Derivate sind in den letzten Jahren intensiv bearbeitet worden, da vom Bicyclo[3.3.0]octan-System abgeleitete Carbacycline wie z.B. Iloprost bzw. Cicaprost oder andere analoge Isocarbacycline biologisch sehr potente wie auch chemisch und teils auch metabolisch stabile Prostacyclin-Mimetika darstellen.
Es wurde überraschenderweise gefunden, daß durch die Einführung eines geeigneten Restes in Position 9 (Prostaglandinzählweise) chemisch und metabolisch stabile Carbacyclin-Analoga erhalten werden, die zusätzlich in der Lage sind, die pharmakologischen Eigenschaften des instabilen Thromboxan-A₂ (TXA₂) bzw. PGH₂ sowie seiner stabilen Analoga wie z.B. U46619 oder U44069 zu antagonisieren.
Die Verbindungen dieser Erfindung stellen deshalb wertvolle Hilfsmittel zur selektiven Therapie von Erkrankungen, die auf einen Mangel an körpereigenem PGI₂ und/oder Überschuß an TXA₂ bzw. PGH₂ zurückzuführen sind, dar.
Die Erfindung betrifft 9-substituierte Bicyclo[3.3.0]octan-Derivate der Formel I, 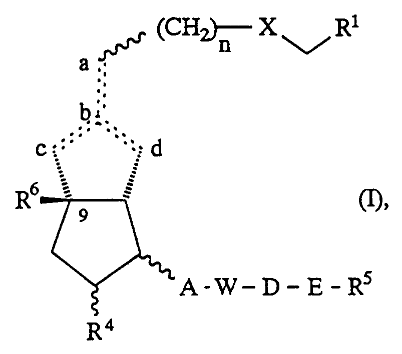
sowie deren Enantiomere,
worin
zwischen den Kohlenstoffatomen der Zentren a-b oder b-c oder b-d maximal eine Doppelbindung liegt,
R¹ 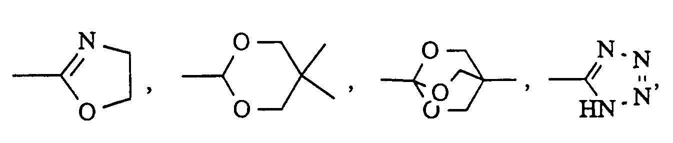
COOR², wobei R² Wasserstoff oder gegebenenfalls durch Halogen, Phenyl, C₁-C₄-Alkoxy oder Di-(C₁-C₄)-alkylamino substituiertes C₁-C₁₀-Alkyl, C₃-C₁₀-Cycloalkyl, C₇-C₁₆-Aralkyl, durch Y substituiertes Phenacyl oder C₆-C₁₂-Aryl oder einen 5- oder 6- gliedrigen heterocyclischen Rest mit wenigstens einem N-, O- oder S-Atom bedeuten kann, oder -CONHR³ mit R³ in der Bedeutung Wasserstoff, C₁-C₁₀-Alkanoyl oder C₁-C₁₀-Alkansulfonyl sein kann,
X eine CH₂-Gruppe, ein Sauerstoffatom oder eine O-CH₂-CH₂-Gruppe,
n 0 bis 3,
R⁴ ein Wasserstoffatom, Halogen, eine freie oder funktionell abgewandelte Hydroxygruppe, wobei die OH-Gruppe α- oder β-ständig sein kann,
R⁶ -(CH₂)_{q}-R⁷ oder ―C≡C―(CH₂)_{q}―R⁷,
q 1 bis 5,
R⁷ 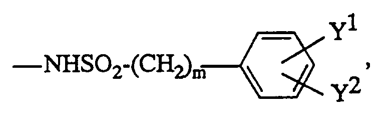
m 0 bis 2
A eine cis oder trans CH=CH- oder eine ―C≡C―Gruppe,
W eine 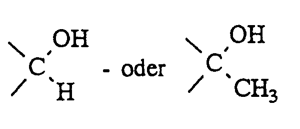
-Gruppe, wobei die OH-Gruppe α- oder β-ständig sein kann,
D und E zusammen eine Bindung oder
D eine Bindung oder C₁-C₁₀-Alkylen,
E eine Bindung, eine ―C≡C―Gruppe, eine -CR⁹=CR¹⁰- Gruppe, wobei R⁹ und R¹⁰ gleich oder verschieden sind und Wasserstoff, Chlor oder Brom, oder eine C₁-C₅-Alkylgruppe bedeuten, oder

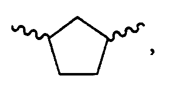

R⁵ ein Wasserstoffatom, durch Y substituiertes C₁-C₁₀-Alkyl, C₃-C₁₀-Cycloalkyl oder C₆-C₁₂-Aryl,
Y¹ und Y² gleich oder verschieden sind und Y bedeuten,
Y Wasserstoff, Halogen, N₃, NH₂, CN, CF₃, OR⁸, NO₂, COOR⁸ oder C₁-C₁₀-Alkyl,
R⁸ Wasserstoff, C₁-C₁₀-Alkyl, gegebenenfalls durch Halogen substituiertes C₆-C₁₂-Aryl oder C₇-C₁₆-Aralkyl sein kann und, falls R² Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

Die Definition 5- oder 6-gliedriger heterocyclischer Rest betrifft Heterocyclen, die wenigstens ein Heteroatom, vorzugsweise Stickstoff, Sauerstoff oder Schwefel enthalten. Beispielsweise seien genannt 2-Furyl, 3-Furyl, 2-Thienyl, 3-Thienyl, 2-Pyridyl, 3-Pyridyl, 4-Pyridyl.

Als Alkylgruppen R², R⁵, R⁸ und Y sind gerad- oder verzweigtkettige Alkylgruppen mit 1 - 10 C-Atomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Die Alkylgruppen R², R⁵, R⁸ und Y können substituiert sein durch Halogenatome, Hydroxygruppen, C₁-C₄-Alkoxygruppen, C₆-C₁₂-Alylgruppen, die durch Halogen substituiert sein können, Di-(C₁-C₄)-Alkylamine und Tri-(C₁-C₄)-Alkylammonium. Bevorzugt sind solche Alkylgruppen, die einfach substituiert sind.
Als Substituenten seien beispielsweise genannt Fluor-, Chlor- oder Bromatome, Phenyl, Dimethylamino, Diethylamino, Methoxy, Ethoxy.
Als bevorzugte Alkylgruppen R², R⁵, R⁸ und Y sind solche mit 1 - 5 C-Atomen, wie z. B. Methyl, Ethyl, Propyl, Isobutyl, Butyl, Chlorethyl, Hydroxyethyl und 1- und 2-Hydroxypropyl zu nennen.

Als Alkylengruppen D sind gerad- oder verzweigtkettige Alkylengruppen mit 1 - 10 C-Atomen zu betrachten, wie beispielsweise Methylen, Ethylen, Propylen, Isopropylen, Butylen, Isobutylen, tert.-Butylen, Pentylen, Isopentylen, Neopentylen, Heptylen, Hexylen, Decylen.
Die Alkylengruppen D können substituiert sein durch Halogenatome, Hydroxygruppen, C₁-C₄-Alkoxygruppen, C₆-C₁₂-Arylgruppen, die durch Halogen substituiert sein können, Di-(C₁-C₄)-Alkylamine und Tri-(C₁-C₄)-Alkylammonium. Bevorzugt sind solche Alkylengruppen, die nicht oder einfach substituiert sind.
Als Substituenten seien beispielsweise genannt Fluor-, Chlor- oder Bromatome, Phenyl, Dimethylamino, Diethylamino, Methoxy, Ethoxy.
Als bevorzugte Alkylengruppen D sind solche mit 1 - 5 C-Atomen, wie z. B. Methylen, Ethylen, Propylen, Isopropylen, Isobutylen, Butylen, Chlorethylen, Hydroxyethylen und 1- und 2-Hydroxypropylen zu nennen.

Als Alkylgruppen R⁹ und R¹⁰ kommen Methyl, Ethyl, Propyl, Isopropyl, Butyl, Isobutyl, Pentyl und Isopentyl in Betracht. Bevorzugt sind Methyl und Ethyl.

Als Arylgruppen R², R⁵ und R⁸ kommen beispielsweise in Betracht: Phenyl, Diphenyl, 1-Naphthyl und 2-Naphthyl, die substituiert sein können durch 1 - 3 Halogenatome, eine Phenylgruppe, 1 - 3 Alkylgruppen mit jeweils 1 - 4 C-Atomen eine Chlormethyl-, Fluormethyl-, Carboxyl-, C₁-C₄-Alkoxy- oder Hydroxygruppe.
Bevorzugt ist die Substitution in 3- und 4-Stellung am Phenylring zum Beispiel durch Fluor, Chlor, C₁-C₄-Alkoxy oder Trifluormethyl oder in 4-Stellung durch Hydroxy.

Die Cycloalkylgruppen R² und R⁵ können im Ring 3-10, vorzugsweise 3-6 Kohlenstoffatome enthalten. Die Ringe können durch Alkylgruppen mit 1-4 Kohlenstoffatomen substituiert sein. Beispielsweise seien genannt Cyclopropyl, Cyclobutyl, Cyclopentyl, Cyclohexyl, Methylcyclopentyl, Methylcyclohexyl.

Die C₇-C₁₆ Aralkylgruppen in R² und R⁸ können im Ring 6 bis 14 C-Atome enthalten, bevorzugt 6 bis 10 (Phenyl oder Naphtyl) und in der Alkylkette 1 bis 4, bevorzugt 1 bis 2 C-Atome. Bevorzugte Aralkylreste sind z.B. Benzyl, Phenylethyl, 1-Phenylethyl, 1-(2)-Naphthylmethyl bzw. 1-(2)-Naphthylethyl.

Die Hydroxygruppen in R⁴, Y und W können funktionell abgewandelt sein, beispielsweise durch Veretherung oder Veresterung, wobei die freien oder abgewandelten Hydroxygruppen in R⁴ und W jeweils α- oder β-ständig sein können, wobei freie Hydroxygruppen bevorzugt sind.

Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, tert-Butyldimethylsilyl-, tert.-Butyldiphenylsilyl-, Tribenzylsilylrest. Als Acylreste kommen z.B. Acetyl, Propionyl, Butyryl, Benzoyl in Frage.

Halogen in den Definitionen für R², R⁴, R⁸ und Y bedeutet Fluor, Chlor, Brom und Iod.

Die Reste "C₁-C₁₀-Alkanoyl" oder "C₁-C₁₀-Alkansulfonyl" für R³ entsprechen den bereits genannten Alkylgruppen gleicher Länge mit dem Unterschied, daß sie an eine Carboxylgruppe gebunden sind. Bevorzugt sind C₁-C₄-Alkanoyl bzw. -Alkansulfonyl.

Zur Salzbildung mit den freien Säuren (R² = H) sind anorganische und organische Basen geeignet, wie sie dem Fachmann zur Bildung physiologisch verträglicher Salze bekannt sind. Beispielsweise seien genannt: Alkalihydroxide, wie Natrium- oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w.

Bevorzugt werden die Verbindungen der Formel I, in denen
R¹ die Gruppen COOR² oder CONHR³,
R⁴ Wasserstoff, Hydroxyl oder Halogen,
R² Wasserstoff oder gegebenenfalls durch Halogen substituiertes C₇-C₁₆-Aralkyl,
C₅-C₆-Cycloalkyl, C₁-C₁₀-Alkyl,
R³ C₁-C₇-Alkanoyl, C₆-C₁₂-Arylsulfonyl, C₁-C₇-Alkansulfonyl, bedeuten.

Besonders bevorzugt werden die Verbindungen der Formel I, in denen
R¹ die Gruppen COOR²,
R⁴ Wasserstoff oder Hydroxyl,
R² Wasserstoff oder Methyl,
R³ Methansulfonyl,
bedeuten.

Die Erfindung betrifft ferner Verfahren zur Herstellung von 9-substituierten Bicyclo[3.3.0]octan-Derivaten der Formel I, dadurch gekennzeichnet, daß man die Hydroxy-Verbindung der Formel II zu einem Aldehyd der Formel III oxidiert und mit einer Verbindung der Formel IV in eine Verbindung der Formel V überführt, 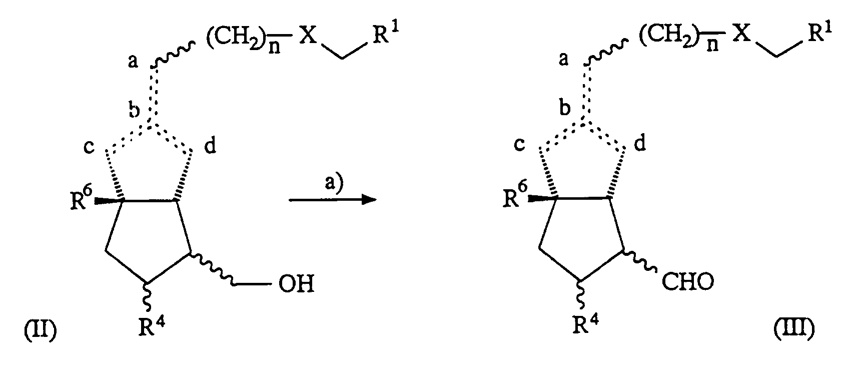 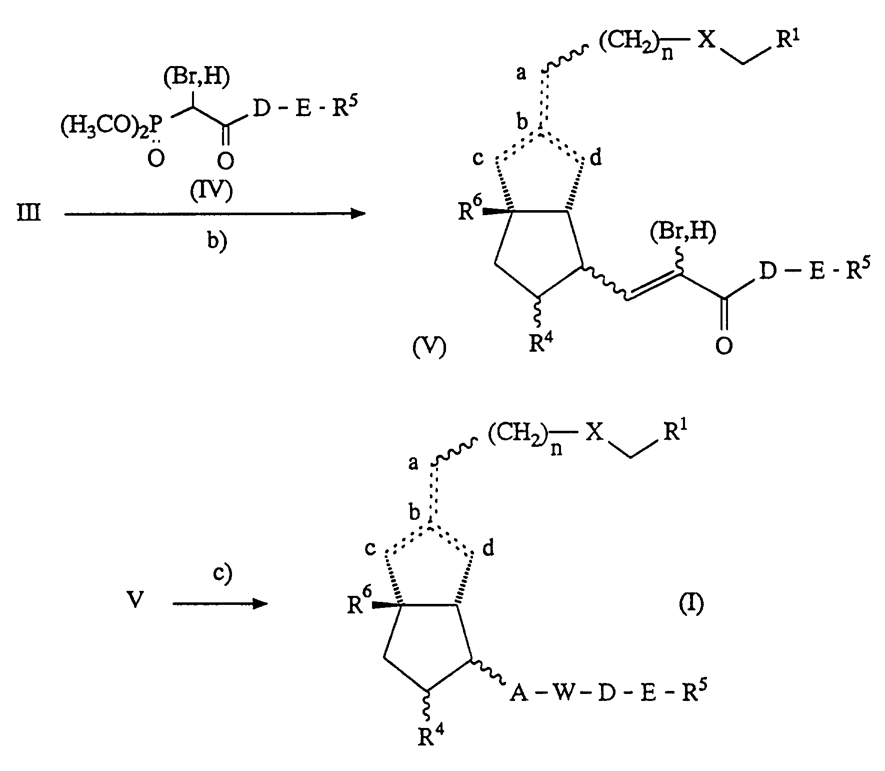
das gebildete Keton reduziert und gegebenenfalls Bromwasserstoff eliminiert, wobei a-b, b-c, b-d, R⁴, R⁵, R⁶, A, W, D, E, n, X, Y¹, Y² und Hal die oben angegebenen Bedeutungen haben und R¹ eine -COOR²-Estergruppe mit R² in der mit Ausnahme von Wasserstoff oben angegebenen Bedeutung darstellt, mit physiologisch verträglichen Basen in deren Salze überführt, mit α-, β- oder γ- Cyclodextrin zu einem Clathrat umwandelt oder mit Liposomen verkapselt.

Die Reaktionsbedingungen der vorstehenden Verfahrensstufen sind:
a) II ⇒ III
   Die Oxidation von Verbindungen der Formel II zu Verbindungen der Formel III erfolgt nach bekannten Verfahren, wie z.B. nach dem von Swern, Collins sowie unter Verwendung von Pyridiniumdichromat bzw. -chlorochromat in Lösungsmitteln wie Dichlormethan, Diethylether, Tetrahydrofuran, Benzol oder Toluol bei -80°C bis -50°C (Swern) bzw. bis +30°C (bei den übrigen Oxidationen) innerhalb von 10 Minuten bis 8 Stunden.
b) III ⇒ V
   Die Umsetzung der Verbindungen der Formel III mit Verbindungen der Formel IV zu Verbindungen der Formel V erfolgt nach den dem Fachmann bekannten Verfahren in einem inerten Lösungsmittel wie beispielsweise Dimethoxyethan unter Verwendung eines Deprotonierungsmittels wie z.B. Natriumhydrid bei -50°C bis +50°C (bevorzugt bei -10°C bis +25°C) in 1 bis 15 Stunden.
c) V ⇒ I
   Die Umsetzung der Verbindungen der Formel V zu Verbindungen der Formel I erfolgt mit einem Reduktionsmittel wie beispielsweise Natriumborhydrid in alkoholischer (bevorzugt methanolischer) Lösung bei -60°C bis -20°C nach den dem Fachmann bekannten Verfahren. Nach dem Freisetzen eventuell geschützter Hydroxylgruppen wird gegebenenfalls, wie in Beispiel 5a beschrieben, Bromwasserstoff eliminiert.

Die Freisetzung der funktionell abgewandelten Hydroxygruppen R⁴, W und Y erfolgt nach den dem Fachmann bekannten Methoden. Beispielsweise wird die Abspaltung von Etherschutzgruppen in einer wässrigen Lösung einer organischen Säure, wie z.B. Essigsäure, Propionsäure, Zitronensäure u.a. oder in einer wässrigen Lösung einer anorganischen Säure, wie z.B. Salzsäure, oder im Falle von Tetrahydropyranylethern unter Verwendung von Pyridinium-p-Toluolsulfonat, vorzugsweise in Alkoholen als Lösungsmittel oder unter Verwendung von wasserfreiem Magnesiumbromid, vorzugsweise in Diethylether als Lösungsmittel durchgeführt.
Zur Verbesserung der Löslichkeit wird bei Verwendung wässrig-saurer Reaktionsbedingungen zweckmäßigerweise ein mit Wasser mischbares inertes Lösungsmittel zugesetzt. Als geeignet erweisen sich z.B. Alkohole wie Methanol und Ethanol, Ether wie Dimethoxyethan, Dioxan und Tetrahydrofuran, wobei Tetrahydrofuran bevorzugt angewendet wird.

Die Abspaltung der Silyletherschutzgruppen erfolgt beispielsweise mit Tetrabutylammoniumfluorid nach den dem Fachmann bekannten Methoden. Als Lösungsmittel sind beispielsweise Tetrahydrofuran, Diethylether, Dioxan, Methylenchlorid etc. geeignet. Die Abspaltung wird vorzugsweise bei Temperaturen zwischen 20°C und 80°C durchgeführt.

Die Verseifung der Acylgruppen und Carbacyclinester wird nach den dem Fachmann bekannten Methoden durchgeführt, wie beispielsweise mit basischen Katalysatoren wie z.B. mit Alkali- oder Erdalkali-carbonaten oder -hydroxiden in einem Alkohol oder der wässrigen Lösung eines Alkohols. Als Alkohole kommen aliphatische Alkohole wie z.B. Methanol, Ethanol, Butanol etc. in Betracht, vorzugsweise jedoch Methanol. Als Alkalicarbonate und -hydroxide seien Lithium-, Natrium- und Kaliumsalze genannt. Bevorzugt sind die Lithium- und Kaliumsalze. Als Erdalkalicarbonate und -hydroxide eignen sich beispielsweise Calciumcarbonat, Calciumhydroxid und Barium-carbonat. Die Umsetzung erfolgt allgemein bei -10°C bis +70°C, vorzugsweise jedoch bei +25°C.

Die Einführung der Estergruppe CO₂R² für R¹ bzw. CO₂R⁸ für Y, bei welcher R² bzw. R⁸ eine Alkylgruppe mit 1-10-C-Atomen darstellt, erfolgt nach den dem Fachmann bekannten Methoden. Die Carboxyverbindungen (R² = H bzw. R⁸ = H) werden beispielsweise mit Diazokohlenwasserstoffen in an sich bekannter Weise umgesetzt. Die Veresterung mit Diazokohlenwasserstoffen erfolgt z.B. dadurch, daß eine Lösung des Diazokohlenwasserstoffes in einem inerten Lösungsmittel, vorzugsweise in Diethylether, mit der Carboxyverbindung, gelöst in dem gleichen oder in einem anderen ebenfalls inerten Lösungsmittel, wie z.B. Methylenchlorid, vermischt wird. Nach beendeter Umsetzung in 1 bis 60 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389-394 (1954)].

Die Einführung der Estergruppe CO₂R² für R¹ bzw. CO₂R⁸ für Y, bei welcher R² bzw. R⁸ eine substituierte oder unsubstituierte Arylgruppe darstellt, erfolgt nach den dem Fachmann bekannten Methoden. Beispielsweise werden die Carboxyverbindungen mit den entsprechenden Arylhydroxyverbindungen mit Dicyclohexylcarbodiimid in Gegenwart einer geeigneten Base wie z.B. Pyridin, DMAP, Triethylamin, in einem inerten Lösungsmittel wie z.B. Methylenchlorid, Ethylenchlorid, Chloroform, Essigsäureethylester, Tetrahydrofuran, vorzugsweise jedoch mit Chloroform umgesetzt. Die Reaktion wird bei Temperaturen zwischen -30°C und +50°C, vorzugsweise bei +10°C, durchgeführt.

Die Carbacyclinderivate der Formel I mit R² bzw. R⁸ in der Bedeutung eines Wasserstoffatomes können mit geeigneten Mengen der entsprechenden anorganischen Basen unter Neutralisierung in Salze überführt werden. Beispielsweise erhält man beim Lösen der entsprechenden Säuren in Wasser, welches stöchiometrische Mengen der Base enthält, nach Abdampfen des Wassers oder nach Zugabe eines mit Wasser mischbaren Lösungsmittels, z.B. Alkohol oder Aceton, das feste anorganische Salz.

Die Herstellung der Aminsalze erfolgt in üblicher Weise. Dazu löst man die Säure in einem geeigneten Lösungsmittel, wie z.B. Ethanol, Aceton, Diethylether oder Benzol und setzt 1 bis 5 Äquivalente des jeweiligen Amins dieser Lösung zu. Dabei fällt das Salz gewöhnlich in fester Form an oder wird nach Verdampfen des Lösungsmittels in üblicher Weise isoliert.

Die funktionelle Abwandlung der freien Hydroxygruppen erfolgt nach den dem Fachmann bekannten Methoden. Zur Einführung der Etherschutzgruppen wird beispielsweise mit Dihydropyran oder Methylvinylether in Methylenchlorid oder Chloroform unter Verwendung katalytischer Mengen eines sauren Kondensationsmittels wie z.B. p-Toluolsulfonsäure, umgesetzt. Der jeweilige Enolether wird im Überschuß, vorzugsweise in der 1,2- bis 10-fachen Menge des theoretischen Bedarfs, zugesetzt. Die Umsetzung erfolgt normalerweise bei -10°C bis +30°C und ist nach 2 bis 45 Minuten beendet.

Zur Einführung von Silyletherschutzgruppen wird beispielsweise mit t-Butyl-diphenylchlorsilan oder t-Butyl-dimethylchlorsilan in Dimethylformamid unter Verwendung einer Base wie z.B. Imidazol, umgesetzt. Das jeweilige Silylchlorid wird im Überschuß, vorzugsweise in der 1,05- bis 4-fachen Menge des theoretischen Bedarfs, zugesetzt. Die Umsetzung erfolgt normalerweise bei 0°C bis 30°C und ist nach 1 bis 24 Stunden beendet.

Die Einführung der Acylschutzgruppen erfolgt, indem man eine Verbindung der Formel I in an sich bekannter Weise mit einem Carbonsäurederivat, wie z.B. Säurechlorid, Säureanhydrid etc., umsetzt.

Die Clathrate mit α-, β- oder γ -Cyclodextrin werden analog der Vorschrift in WO 87/05294 erhalten. Bevorzugt wird β-Cyclodextrin verwendet.

Liposomen werden nach dem in "Pharmazie in unserer Zeit 11, 98 (1982)" beschriebenen Herstellungsverfahren hergestellt.

Alle stereoisomeren Formen gehören ebenfalls zum Gegenstand der Erfindung.

### Biologische Wirkung und Anwendungsbereich der neuen TXA₂-Antagonisten:

Die Verbindungen dieser Erfindung eignen sich zur Therapie von Erkrankungen des cardiovaskulären Systems, des Magens, des Pankreas, der Leber und der Niere. Sie wirken blutdrucksenkend und bronchodilatorisch. Sie sind hervorragend geeignet zur Hemmung der Thrombozytenaktivierung. Folglich stellen die neuen TXA₂-Antagonisten der Formel I wertvolle pharmazeutische Wirkstoffe dar. Darüber hinaus zeichnen sich die Verbindungen durch eine Prostacyclin-mimetische Wirkkomponente, durch höhere Selektivität, eine wesentlich längere Wirksamkeit und eine größere Stabilität verglichen mit ähnlichen TXA₂-Antagonisten aus.
Die neuen TXA₂-Antagonisten besitzen die für diese Verbindungsklasse typischen Eigenschaften, wie z.B. Senkung des peripheren arteriellen, des koronaren und des pulmonalen Gefäßwiderstandes, Senkung des pulmonalen Blutdrucks, Erniedrigung des systemischen Blutdrucks ohne zugleich Schlagvolumen und koronare Durchblutung zu senken, Förderung der Nierendurchblutung und der Durchblutung anderer peripherer Organe, Erhöhung der cerebralen Durchblutung, Inhibierung der Thrombozytenaktivierung und Auflösung von Thromben, Inhibierung der Bronchokonstriktion, Inhibierung der Magensäuresekretion, Zytoprotektion des Herzens, der Magen- und Darmschleimhaut, der Leber, Zytoprotektion im Pankreas und in der Niere sowie antiallergische Eigenschaften. Daher sind die neuen TXA₂-Antagonisten prinzipiell geeignet zur Behandlung des Schlaganfalles, der Prophylaxe und Therapie koronarer Herzerkrankungen, zum Beispiel der Koronarthrombose, zur Behandlung des Herzinfarktes, peripherer Arterienerkrankungen, zur Prophylaxe und Therapie bei anderen thromboembolischen Erkrankungen und bei Arteriosklerose, bei ischämischen Attacken des ZNS-Systems und anderer Durchblutungsstörungen des Gehirns wie z.B. der Migräne, zur Behandlung der Hypertonie und zur Behandlung von Krankheiten, die mit einer Erhöhung des pulmonalen Gefäßwiderstandes einhergehen wie z.B. der pulmonalen Hypertonie und zur Therapie des Schocks, des Asthmas und der allergischen Rhinitis. Sie können ferner eingesetzt werden zur Inhibierung von Geburtswehen und zur Behandlung von Schwangerschaftstoxikosen.
Die neuen TXA₂-Antagonisten können außerdem Anwendung finden zur Verbesserung der Organfunktion nach Transplantation, zum Beispiel bei der Nierentransplantation, zur Verhinderung von Abstoßungsreaktionen, an Stelle von Heparin oder als Adjuvans bei der Dialyse oder Hämofiltration und bei der Konservierung von Blutplasmakonserven, zum Beispiel von Blutplättchenkonserven.
Die neuen TXA₂-Antagonisten besitzen eine antimetastatische Wirkung und antiproliferative Eigenschaften. Sie sind prinzipiell geeignet zur Behandlung von Neoplasien. Die neuen TXA₂-Antagonisten können in Kombination, zum Beispiel mit Carbacyclinen, Prostacyclin und seinen Analoga, 7-Oxo-prostacyclinen, Prostaglandinen und deren Derivate und 6-Oxo-PGE₁- und 6-Oxo-9-Fluor-Prostaglandin-Derivaten, mit TXA₂-Synthetase-Inhibitoren, mit Phosphodiesterase-Inhibitoren, mit Antagonisten und Rezeptorantagonisten verschiedener Thrombozytenstimulatoren (z.B. ADP, Thrombin, Collagen, PAF, Adrenalin, Serotonin, Fibrinogen), mit Calciumantagonisten, mit Fibrinolytika und Thrombolytika, z.B. t-PA, Streptokinase, mit Heparin und anderen Antikoagulanzien, mit Cyclooxygenasehemmern, z.B. Acetylsalicylsäure, mit Hemmstoffen der Lipoxy-genasen sowie Antagonisten von Lipoxygenaseprodukten, mit Vasodilatatoren wie z.B. Nitroverbindungen, mit Antihypertensiva wie z.B. β-Blockern oder mit Diuretika Verwendung finden.
Die Dosis der Verbindungen ist 0,1-1000 mg/Tag, bevorzugt 0,1-500 mg/Tag, auch in mehreren Teildosierungen, wenn sie am menschlichen Patienten verabreicht werden. Die Einheitsdosis für den pharmazeutisch akzeptablen Träger beträgt 0,1-100 mg. Für die parenterale Verabreichung werden sterile, injizierbare wässrige oder ölige Losungen benutzt. Für die orale Applikation sind beispielsweise Tabletten, Dragees oder Kapseln geeignet.
Die Erfindung betrifft damit auch Arzneimittel auf Basis der Verbindungen der allgemeinen Formel I und üblicher Hilfs- und Trägerstoffe.
Die erfindungsgemäßen Wirkstoffe sollen in Verbindung mit den in der Galenik bekannten und üblichen Hilfsstoffen z.B. zur Herstellung von Blutdrucksenkern dienen.
Der Einheitsdosisberich für die Ampulle ist 0,1-100 mg, für die Tablette 0,1-100 mg.

### Beispiel 1:

### 5-[(3E/Z,1R,5R,6R,7R)-1-(4-Chlorphenylsulfonylaminomethyl)-6-[(1E,3S,4S)-3-hydroxy-4-methyl-1-nonen-6-inyl]-7-hydroxy-bicyclo[3.3.0]oct-3-yliden]-pentansäure:

Die Lösung von 58,3 mg (99 µmol) der nach Beispiel 1a dargestellten Verbindung A in 2,5 ml Methanol versetzt man mit 2 ml einer 5%igen Lithiumhydroxidlösung und rührt 2,5 Stunden bei 23°C. Durch Zugabe von gesättigter Zitronensäure wird angesäuert, mit Wasser verdünnt und mehrfach mit Chloroform extrahiert. Man wäscht mit Wasser, gesättigter Natriumchloridlösung und trocknet über Magnesiumsulfat. Den nach Filtration und Lösungsmittelabzug erhaltenen Rückstand reinigt man durch Chromatographie an 3 analytischen Dünnschichtplatten. Als Laufmittel dient ein Gemisch aus Dichlormethan und Ethanol, als Elutionsmittel ein Gemisch aus Chloroform und Isopropanol. Isoliert werden 53 mg (92 µmol, 93%) der Titelverbindung als farbloses Öl.
¹H-NMR (CDCl₃): δ= 0,92 (d,3H), 1,12 (t,3H), 1,38 (m,1H), 1,6-2,4 (m,20H), 2,75 (d,2H), 3,5-5,0 (s,1H), 3,78 (m,1H), 3,93 (m,1H), 5,2 (m,1H), 5,5 (m,2H), 5,96 (m,1H), 7,48 (d,2H), 7,8 (d,2H).

### Beispiel 1a:

### 5-[(3E/Z,1R,5R,6R,7R)-1-(4-Chlorphenylsulfonylaminomethyl)-6-[(1E,3S,4S)-3-hydroxy-4-methyl-1-nonen-6-inyl]-7-hydroxy-bicyclo[3.3.0]oct-3-yliden]-pentansäuremethylester (A) und 5-[(3E/Z,1R,5R,6R,7R)-1-(4-Chlorphenylsulfonylaminomethyl)-6-[(1E,3R,4S)-3-hydroxy-4-methyl-1-nonen-6-inyl]-7-hydroxy-bicyclo[3.3.0]oct-3-yliden]-pentansäuremethylester (B):

Die Lösung von 160 mg (237 µmol) der nach Beispiel 1b dargestellten Verbindung in 11 ml wasserfreiem Ethanol versetzt man mit einer Mikrospatelspitze Pyridinium-p-toluolsulfonat und erwärmt unter einer Atmosphäre aus trockenem Argon 2,5 Stunden auf 55°C. Man engt ein, nimmt in Dichlormethan auf und reinigt durch Chromatographie an 7 analytischen Dünnschichtplatten. Als Lauf- und Elutionsmittel dient Ethylacetat. Isoliert werden 51,5 mg (87 µmol, 37%) einer unpolaren Komponente, der man die Struktur B zuordnet, sowie 58,3 mg (99 mmol, 42%) einer polaren Komponente, der man die Struktur A zuordnet.
IR (Film) von A: 3600-3100, 3280, 3050, 2960, 2910, 2850, 1725, 1585, 1430, 1330, 1265, 1160, 1090, 1065, 1010, 970, 825, 735, 700 und 620 cm⁻¹.
IR (Film) von B: 3600-3100, 3270, 3050, 2960, 2910, 2850, 1725, 1585, 1435, 1330, 1265, 1160, 1090, 1080, 1065, 1010, 970, 825, 750, 735, 700 und 615 cm⁻¹.

### Beispiel 1b:

### 5-[(3E/Z,1R,5R,6R,7R)-1-(4-Chlorphenylsulfonylaminomethyl)-6-[(1E,3RS,4S)-3-hydroxy-4-methyl-1-nonen-6-inyl]-7-(tetrahydropyran-2-yloxy)-bicyclo[3.3.0]oct-3-yliden] -pentansäuremethylester:

Die Lösung von 159 mg (235 µmol) der nach Beispiel 1c dargestellten Verbindung löst man in 8,5 ml wasserfreiem Methanol, kühlt unter einer Atmosphäre aus trockenem Aregon auf -50⁰C und versetzt portionsweise mit insgesamt 22 mg Natriumborhydrid. Man läßt noch 30 Minuten reagieren, zersetzt überschüssiges Reduktionsmittel durch Zugabe von 40 µl Aceton, versetzt mit Wasser und extrahiert mehrfach mit Diethylether. Man wäscht mit Wasser, gesättigter Natriumchloridlösung und trocknet über Magnesiumsulfat. Den nach Filtration und Lösungsmittelabzug erhaltenen Rückstand von 160 mg (235 µmol, 100%) setzt man ohne Reinigung weiter um.
IR (Film): 3600-3100, 3270, 3050, 2960, 2920, 2850, 1730, 1585, 1430, 1330, 1260, 1160, 1090, 1065, 1010, 970, 865, 825, 735, 700 und 620 cm⁻¹.

### Beispiel 1c:

### 5-[(3E/Z,1R,5R,6R,7R)-1-(4-Chlorphenylsulfonylaminomethyl)-6-[(1E,4S)-3-oxo-4-methyl -1-nonen-6-inyl]-7-(tetrahydropyran-2-yloxy)-bicyclo[3.3.0]oct-3-yliden]-pentansäuremethylester:

Zu der Aufschlämmung von 25 mg Natriumhydrid-Dispersion (80%ig) in 4 ml wasserfreiem Dimethoxyethan tropft man unter einer Atmosphäre aus trockenem Argon die Lösung von 227 mg Dimethyl-(2-oxo-3S-methyl-oct-5-inyl)-phosphonat in 3 ml wasserfreiem Dimethoxyethan und rührt 20 Minuten bei 23°C. Anschließend versetzt man mit der Lösung von 440 mg (ca. 922 µmol) des nach Beispiel 1d dargestellten Rohaldehyds in 4 ml Dichlormethan und rührt 2 Stunden. Nach Zugabe von weiteren 23 mg Natriumhydrid-Dispersion läßt man noch 1 Stunde reagieren, säuert durch Zugabe von gesättigter Citronensäure an, versetzt mit Wasser und extrahiert mehrfach mit Diethylether. Die vereinigten organischen Extrakte wäscht man mit Wasser und gesättigter Natriumchloridlösung, trocknet über Magnesiumsulfat und reinigt den nach Lösungsmittelabzug erhaltenen Rückstand durch Chromatographie an ca. 50 ml feinem Kieselgel mit einem Gradientensystem aus n-Hexan und Ethylacetat. Isoliert werden 159 mg (235 µmol, 31%) der Titelverbindung als farbloses Öl.
¹H-NMR (CDCl₃): δ= 1,08 (t,3H), 1,19 (d,3H), 1,35-1,75 (m,11H), 1,8-2,5 (m,14H), 2,75-3,0 (m,2H), 3,43 (m,1H), 3,67 (s,3H), 3,65-4,0 (m,2H), 4,48-4,6 (m,1H), 5,02 (m,1H), 5,21 (t,1H), 6,2 (2d,1H), 6,78 (2dd,1H), 7,5 (d,2H), 7,79 (d,2H).

### Beispiel 1d:

### 5-[(3E/Z,1R,5R,6R,7R)-1-(4-Chlorphenylsulfonylaminomethyl)-6-formyl-7-(tetrahydropyran-2-yloxy)-bicyclo[3.3.0]oct-3-yliden]-pentansäuremethylester:

Zu der unter einer Atmosphäre aus trockenem Argon bei -70°C vorgelegten Lösung von 130 µl frisch destilliertem Oxalylchlorid in 2,6 ml wasserfreiem Dichlormethan tropft man die Lösung von 237 µl Dimethylsulfoxid in 1 ml Dichlormethan, läßt 25 Minuten reagieren und versetzt anschließend mit der Lösung von 500 mg (899 µmol) des nach Beispiel 1e dargestellten Alkohols in 3,1 ml Dichlormethan. Man läßt 2,5 Stunden reagieren, versetzt mit 0,4 ml Triethylamin, gießt in Eiswasser und extrahiert mehrfach mit Dichlormethan. Die vereinigten organischen Extrakte trocknet man über Magnesiumsulfat. Den nach Filtration und Lösungsmittelabzug erhaltenen Rückstand von 550 mg (max. 899 µmol) setzt man ohne Reinigung weiter um.

### Beispiel 1e:

### 5-[(3E/Z,1R,5R,6S,7R)-1-(4-Chlorphenylsulfonylaminomethyl)-6-hydroxymethyl-7-(tetrahydropyran-2-yloxy)-bicyclo[3.3.0]oct-3-yliden]-pentansäuremethylester:

Die Lösung von 1,56 g (1,96 mmol) der nach Beispiel 1f dargestellten Verbindung in 30 ml wasserfreiem Tetrahydrofuran versetzt man mit 3,8 ml einer 1M Lösung von Tetrabutylammoniumfluorid in Tetrahydrofuran und rührt 1,5 Stunden bei 23°C unter einer Atmosphäre aus trockenem Argon. Man engt ein und reinigt durch Chromatographie an ca. 80 ml feinem Kieselgel mit einem Gradientensystem aus n-Hexan und Ethylacetat. Isoliert werden 1,08 g (1,94 mmol, 99%) der Titelverbindung als farbloses Öl.
IR (Film): 3600-3100, 3270, 3050, 2940, 2860, 1730, 1590, 1435, 1330, 1160, 1075, 1025, 865, 835, 750 und 735 cm⁻¹.

### Beispiel 1f:

### 5-[(3E/Z,1R,5R,6S,7R)-1-(4-Chlorphenylsulfonylaminomethyl)-6-(t-butyldiphenylsilyloxymethyl)-7-(tetrahydropyran-2-yloxy)-bicyclo[3.3.0]oct-3-yliden]-pentansäuremethylester:

Zu der Emulsion aus 5,64 g Carboxybutyltriphenylphosphoniumbromid in 5 ml wasserfreiem Tetrahydrofuran und 12 ml wasserfreiem Dimethylsulfoxid gibt man innerhalb einer Stunde portionsweise insgesamt 3 g fein pulverisiertes Kalium-tert.-butanolat. Man rührt so lange, bis eine klare rote Lösung entsteht, tropft zügig die Lösung von 1,61 g (2,31 mmol) der nach Beispiel 1g dargestellten Verbindung in 5 ml wasserfreiem Tetrahydrofuran zu und läßt 1,5 Stunden bei 50°C unter einer Atmosphäre aus trockenem Argon reagieren. Man gießt in Eiswasser, stellt durch Zugabe einer gesättigten Zitronensäurelösung einen pH-Wert von 4-5 ein und extrahiert mehrfach mit Dichlormethan. Man wäscht mit Wasser und gesättigter Natriumchloridlösung, trocknet über Magnesiumsulfat, filtriert und engt ein. Den erhaltenen Rückstand nimmt man in Dichlormethan auf, versetzt bei 3°C mit der etherischen Lösung von Diazomethan, engt nach beendeter Umsetzung ein und reinigt durch Chromatographie an ca. 100 ml feinem Kieselgel mit einem Gradientensystem aus n-Hexan und Ethylacetat. Isoliert werden 1,56 g (1,96 mmol, 85%) der Titelverbindung als farbloses Öl.
IR (Film): 3270, 3060, 2940, 2850, 1730, 1585, 1425, 1330, 1160, 1110, 1025, 865, 820, 735 und 705 cm⁻¹.

### Beispiel 1g:

### (1S,5R,6S,7R)-1-(4-Chlorphenylsulfonylaminomethyl)-6-(t-butyldiphenylsilyloxymethyl)-7-(tetrahydropyran-2-yloxy)-bicyclo[3.3.0]oct-3-on:

Zu der roten Lösung von 4 g Collins-Reagenz in 80 ml wasserfreiem Dichlormethan tropft man zügig die Lösung von 1,66 g (2,38 mmol) der nach Beispiel 1f dargestellten Verbindung in 80 ml wasserfreiem Dichlormethan zu und rührt 0,5 Stunden bei 23°C unter einer Atmosphäre aus trockenem Argon. Man filtriert, wäscht gut mit Dichlormethan nach, engt ein und reinigt den Rückstand durch Chromatographie an ca. 180 ml feinem Kieselgel mit einem Gradientensystem aus n-Hexan und Ethylacetat. Isoliert werden 1,56 g (2,22 mmol, 93%) der Titelverbindung als farbloses Öl.
IR (Film): 3270, 3050, 2940, 2860, 1735, 1585, 1470, 1425, 1335, 1265, 1160, 1110, 1090, 1020, 865, 820, 735 und 705 cm⁻¹.

### Beispiel 1h:

### (1R,3RS,5R,6S,7R)-1-(4-Chlorphenylsulfonylaminomethyl)-6-(t-butyldiphenylsilyloxymethyl)-7-(tetrahydropyran-2-yloxy)-bicyclo[3.3.0]oct-3-ol:

Die Lösung von 1,98 g (3,78 mmol) des nach Beispiel 1i dargestellten Aminoalkohols in 20 ml wasserfreiem Dichlormethan versetzt man mit 0,75 ml Triethylamin, 1,19 g 4-Chlorsulfonsäurechlorid und rührt 3 Stunden bei 23°C unter einer Atmosphäre aus trockenem Argon. Man engt ein und reinigt durch Chromatographie an ca. 80 ml feinem Kieselgel mit einem Gradientensystem aus n-Hexan und Ethylacetat. Isoliert werden 1,66 g (2,38 mmol, 64%) der Titelverbindung als farbloses Öl.
IR (Film): 3600-3100, 3270, 3060, 2940, 2850, 1585, 1470, 1425, 1330, 1260, 1160, 1110, 1080, 1020, 865, 825, 735 und 705 cm⁻¹.

### Beispiel 1i:

### (1R,3RS,5R,6S,7R)-1-Aminomethyl-6-(t-butyldiphenylsilyloxymethyl)-7-(tetrahydropyran-2-yloxy)-bicyclo[3.3.0]oct-3-ol:

Zu der Aufschlämmung von 420 mg Lithiumaluminiumhydrid in 35 ml wasserfreiem Diethylether tropft man innerhalb 30 Minuten die lösung von 1,93 g (3,74 mmol) des nach Beispiel 1k dargestellten Nitrils in 20 ml Diethylether und rührt 1 Stunde bei 23°C unter einer Atmosphäre aus trockenem Argon. Unter Eiskühlung versetzt man nacheinander mit 5 ml Ethylacetat, 420 µl Wasser, 420 µl einer 15%igen Natriumhydroxidlösung, 1,25 ml Wasser und rührt so lange, bis ein feinkörniger Niederschlag entsteht. Man saugt ab, wäscht mit Chloroform nach und engt ein. Isoliert werden 2,08 g (max. 3,74 mmol) der Titelverbindung als farbloses Öl, das ohne Reinigung weiter umgesetzt wird.
IR (Film): 3600-3100, 3070, 3050, 2930, 2850, 1590, 1465, 1425, 1260, 1110, 1075, 1025, 865, 820, 735 und 705 cm⁻¹.

### Beispiel 1k:

### (1S,5R,6S,7R)-1-Cyano-6-(t-butyldiphenylsilyloxymethyl)-7-(tetrahydropyran-2-yloxy)-bicyclo[3.3.0]oct-3-on:

Die Lösung von 2,45 g (4,99 mmol) (5R,6S,7R)-7-(Tetrahydropyran-2-yloxy)-6-tert.-butyl-diphenylsilyloxymethyl-bicyclo[3.3.0]oct-1-en-3-on (Darstellung siehe EP 0 358 290 A) in 30 ml wasserfreiem Toluol versetzt man mit 1,6 g 18-Krone-6, 655 mg Kaliumcyanid, 550 µl Acetoncyanhydrin und rührt bei 23°C unter einer Atmosphäre aus trockenem Argon. Man gießt auf 100 ml Wasser, extrahiert mehrfach mit Diethylether, wäscht die vereinigten organischen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an ca. 80 ml feinem Kieselgel mit einem Gradientensystem aus n-Hexan und Ethylacetat. Isoliert werden 1,57 g (3,04 mmol, 61%) der Titelverbindung als farbloses Öl.
IR (Film): 3070, 3050, 2940, 2860, 2230, 1745, 1590, 1425, 1110, 1080, 1025, 870, 820, 740 und 705 cm⁻¹.

### Beispiel 2:

### 5-[(3E/Z,1R,5R,6R,7R)-1-(4-Chlorphenylsulfonylaminomethyl)-6-[(1E,3R,4S)-3-hydroxy-4 -methyl-1-nonen-6-inyl]-7-hydroxy-bicyclo[3.3.0]oct-3-yliden]-pentansäure:

51,5 mg (86 µmol) der nach Beispiel 1a dargestellten Verbindung B verseift man in Analogie zu Beispiel 1 und isoliert nach Aufarbeitung und Reinigung 44 mg (77 µmol, 89%) der Titelverbindung als farbloses Öl.
¹H-NMR (CDCl₃): δ= 0,95 (d,3H), 1,1 (t,3H), 1,42 (m,1H), 1,6-2,4 (m,20H), 2,75 (m,2H), 3,5-5,0 (s,1H), 3,82 (m,1H), 4,17 (m,1H), 5,2 (m,1H), 5,58 (m,2H), 5,88 (m,1H), 7,48 (d,2H), 7,8 (d,2H).

### Beispiel 3:

### 4-[(3E/Z,1R,5R,6R,7R)-1-(4-Chlorphenylsulfonylaminomethyl)-6-[(1E,3S,4S)-3-hydroxy-4 -methyl-1-nonen-6-inyl]-7-hydroxy-bicyclo[3.3.0]oct-3-yliden]-butansäure:

14,6 mg (25 µmol) der nach Beispiel 3a dargestellten Verbindung B verseift man in Analogie zu Beispiel 1 und isoliert nach Aufarbeitung und Reinigung 13,8 mg (24 µmol, 97%) der Titelverbindung als farbloses Öl.
¹H-NMR (CD₃OD): δ= 0,92 (d,3H), 1,1 (t,3H), 1,38 (m,1H), 1,66 (m,1H), 1,75-2,45 (m,15H), 2,75 (s,2H), 3,72 (m,1H), 3,9 (m,1H), 5,27 (m,1H), 5,48 (m,2H), 7,54 (d,2H), 7,82 (d,2H).

### Beispiel 3a:

### 4-[(3E/Z,1R,5R,6R,7R)-1-(4-Chlorphenylsulfonylaminomethyl)-6-[(1E,3R,4S)-3-hydroxy-4 -methyl-1-nonen-6-inyl]-7-hydroxy-bicyclo[3.3.0]oct-3-yliden]-butansäuremethylester (A) und 4-[(3E/Z,1R,5R,6R,7R)-1-(4-Chlorphenylsulfonylaminomethyl)-6-[(1E,3S,4S)-3-hydroxy-4-methyl-1-nonen-6-inyl]-7-hydroxy-bicyclo[3.3.0]oct-3-yliden]-butansäuremethylester (B):

35 mg (53 µmol) der nach Beispiel 3b dargestellten Verbindung setzt man in Analogie zu Beispiel 1a um und isoliert nach Aufarbeitung und Reinigung 12,2 mg (21 µmol, 40%) einer unpolaren Komponente, der man die Struktur A zuordnete, sowie 14,6 mg (25 µmol, 48%) einer polaren Komponente, der man die Struktur B zuordnete, jeweils als farbloses Öl.
¹H-NMR (CDCl₃) von A: δ= 0,94 (d,3H), 1,12 (t,3H), 1,45 (m,1H), 1,65-2,45 (m,16H), 2,78 (m,2H), 3,66 (s,3H), 3,82 (m,1H), 3,97 (m,1H), 5,18 (m,1H), 5,4 (t,1H), 5,52 (m,2H), 7,5 (d,2H), 7,82 (d,2H).
¹H-NMR (CDCl₃) von B: δ= 0,96 (d,3H), 1,12 (t,3H), 1,47 (m,1H), 1,65-2,45 (m,16H), 2,79 (m,2H), 3,66 (s,3H), 3,82 (m,1H), 4,19 (m,1H), 5,17 (m,1H), 5,25 (t,1H), 5,58 (m,2H), 7,5 (d,2H), 7,82 (d,2H).

### Beispiel 3b:

### 4-[(3E/Z,1R,5R,6R,7R)-1-(4-Chlorphenylsulfonylaminomethyl)-6[(1E,3RS,4S)-3-hydroxy -4-methyl-1-nonen-6-inyl]-7-(tetrahydropyran-2-yloxy)-bicyclo[3.3.0]oct-3-yliden]-butansäuremethylester:

40 mg (61 µmol) der nach Beispiel 3c dargestellten Verbindung setzt man in Analogie zu Beispiel 1b um und isoliert nach Aufarbeitung und Reinigung 35 mg (53 µmol, 87%) der Titelverbindung als farbloses Öl.
IR (Film): 3600-3100, 3270, 3050, 2940, 2850, 1725, 1585, 1430, 1330, 1265, 1160, 1090, 1060, 1010, 970, 865, 825, 750, 735, 700 und 615 cm⁻¹.

### Beispiel 3c:

### 4-[(3E/Z,1R,5R,6R,7R)-1-(4-Chlorphenylsulfonylaminomethyl)-6-[(1E,4S)-3-oxo-4-methyl -1-nonen-6-inyl]-7-(tetrahydropyran-2-yloxy)-bicyclo[3.3.0]oct-3-yliden]-butansäuremethylester:

189 mg (350 µmol) der nach Beispiel 3d dargestellten Verbindung setzt man in Analogie zu Beispiel 1d um und isoliert nach Aufarbeitung und Reinigung 40 mg (61 µmol, 17%) der Titelverbindung als farbloses Öl.
IR (Film): 3270, 3050, 2940, 2870, 2850, 1730, 1690, 1660, 1620, 1585, 1435, 1335, 1265, 1160, 1075, 1030, 975, 865, 835, 750 und 735 cm⁻¹.

### Beispiel 3d:

### 4-[(3E/Z,1R,5R,6R,7R)-1-(4-Chlorphenylsulfonylaminomethyl)-6-formyl-7-(tetrahydropyran-2-yloxy)-bicyclo[3.3.0]oct-3-yliden]-butansäuremethylester:

522 mg (963 µmol) der nach Beispiel 3e dargestellten Verbindung setzt man in Analogie zu Beispiel 1d um und isoliert nach Aufarbeitung 488 mg (904 µmol, 94%) der Titelverbindung, die man ohne Reinigung weiter umsetzt.

### Beispiel 3e:

### 4-[(3E/Z,1R,5R,6S,7R)-1-(4-Chlorphenylsulfonylaminomethyl)-6-hydroxymethyl-7-(tetrahydropyran-2-yloxy)-bicyclo[3.3.0]oct-3-yliden]-butansäuremethylester:

828 mg (1,06 mmol) der nach Beispiel 3f dargestellten Verbindung setzt man in Analogie zu Beispiel 1e um und isoliert nach Aufarbeitung und Reinigung 522 mg (963 µmol, 91%) der Titelverbindung als farbloses Öl.
IR (Film): 3600-3100, 3280, 3050, 2940, 2860, 1730, 1585, 1470, 1435, 1335, 1160, 1090, 1075, 1025, 975, 865, 825, 750 und 735 cm⁻¹.

### Beispiel 3f:

### 4-[(3E/Z,1R,5R,6S,7R)-1-(4-Chlorphenylsulfonylaminomethyl)-6-(t-butyldiphenylsilyloxymethyl)-7-(tetrahydropyran-2-yloxy)-bicyclo[3.3.0]oct-3-yliden]-butansäuremethylester:

1,11 g (1,59 mmol) der nach Beispiel 1g dargestellten Verbindung setzt man in Analogie zu Beispiel 1f unter Verwendung von Carboxypropyltriphenylphosphoniumbromid um und isoliert nach Aufarbeitung, Veresterung und Reinigung 828 mg (1,06 mmol, 67%) der Titelverbindung als farbloses Öl.
IR (Film): 3270, 3050, 2940, 2850, 1730, 1585, 1425, 1330, 1160, 1110, 1025, 865, 815, 735 und 700 cm⁻¹.

### Beispiel 4:

### 4-[(3E/Z,1R,5R,6R,7R)-1-(4-Chlorphenylsulfonylaminomethyl)-6-[(1E,3R,4S)-3-hydroxy-4 -methyl-1-nonen-6-inyl]-7-hydroxy-bicyclo[3.3.0]oct-3-yliden]-butansäure:

12,2 mg (21 µmol) der nach Beispiel 3a dargestellten Verbindung A verseift man in Analogie zu Beispiel 1 und isoliert nach Aufarbeitung und Reinigung 10,5 mg (19 µmol, 88%) der Titelverbindung als farbloses Öl.
¹H-NMR (CD₃OD): δ= 0,98 (d,3H), 1,1 (t,3H), 1,38 (m,1H), 1,65 (m,1H), 1,8-2,4 (m,15H), 2,73 (s,2H), 3,12 (m,1H), 4,02 (m,1H), 5,25 (m,1H), 5,52 (m,2H), 7,58 (d,2H), 7,82 (d,2H).

### Beispiel 5:

### 5-[(3E/Z,1R,5R,6S,7R)-1-(4-Chlorphenylsulfonylaminomethyl)-6-[(3S,4S)-3-hydroxy-4-methyl-nona-1,6-diinyl]-7-hydroxy-bicyclo[3.3.0]oct-3-yliden]-pentansäure:

100 mg (169 µmol) der nach Beispiel 5a dargestellten Verbindung verseift man in Analogie zu Beispiel 1 und isoliert nach Aufarbeitung und Reinigung 92 mg (159 µmol, 94%) der Titelverbindung als farbloses Öl.
¹H-NMR (CDCl₃): δ= 1,03 (d,3H), 1,12 (t,3H), 1,4 (m,1H), 1,6-1,75 (m,2H), 1,88 (m,1H), 1,95-2,4 (m,16H), 2,77 (m,2H), 3,5-4,8 (s,1H), 4,02 (m,1H), 4,34 (m,1H), 5,23 (m,1H), 5,88 (m,1H), 7,49 (d,2H), 7,79 (d,2H).

### Beispiel 5a:

### 5-[(3E/Z,1R,5R,6S,7R)-1-(4-Chlorphenylsulfonylaminomethyl)-6-[(3S,4S)-3-hydroxy-4-methyl-nona-1,6-diinyl]-7-hydroxy-bicyclo[3.3.0]oct-3-yliden]-pentansäuremethylester:

Die Lösung von 153 mg (228 µmol) der nach Beispiel 5b dargestellten Verbindung A in 7 ml wasserfreiem Toluol versetzt man mit 169 mg 18-Krone-6, 141 mg Cäsiumacetat und erhitzt unter einer Atmosphäre aus trockenem Argon 16 Stunden zum Rückfluß. Man reinigt durch Chromatographie an 10 analytischen Dünnschichtplatten. Als Laufmittel dient ein Gemisch aus n-Hexan und Ethylacetat, als Elutionsmittel Ethylacetat. Isoliert werden 100 mg (169 µmol, 74%) der Titelverbindung als farbloses Öl.
¹H-NMR (CDCl₃): δ= 1,06 (d,3H), 1,12 (t,3H), 1,45 (m,1H), 1,6-1,75 (m,4H), 1,85-2,55 (m,16H), 2,82 (m,2H), 3,68 (s,3H), 4,08 (m,1H), 4,37 (m,1H), 5,22 (m,1H), 5,31 (m,1H), 7,5 (d,2H), 7,79 (d,2H).

### Beispiel 5b:

### 5-[(3E/Z,1R,5R,6S,7R)-1-(4-Chlorphenylsulfonylaminomethyl)-6-[(1Z,3S,4S)-2-brom-3-hydroxy-4-methyl-non-1-en-6-inyl]-7-hydroxy-bicyclo[3.3.0]oct-3-yliden]-pentansäuremethylester (A) und 5-[(3E/Z,1R,5R,6S,7R)-1-(4-Chlorphenylsulfonylaminomethyl)-6-[(1Z,3R,4S)-2-brom-3-hydroxy-4-methyl-non-1-en-6-inyl]-7-hydroxy-bicyclo[3.3.0]oct-3-yliden]-pentansäuremethylester (B):

406 mg (538 µmol) der nach Beispiel 5c dargestellten Verbindungen setzt man in Analogie zu Beispiel 1a um und isoliert nach Aufarbeitung und Reinigung 178 mg (265 µmol, 49%) einer unpolaren Komponente, der man die Struktur B zuordnet, sowie 153 mg (228 µmol, 42%) einer polaren Komponente, der man die Struktur A zuordnet, jeweils als farbloses Öl.
IR (Film) von A und B: 3700-3100, 3280, 3050, 2950, 2870, 1725, 1585, 1430, 1330, 1260, 1160, 1090, 1010, 825, 735 und 700 cm⁻¹.

### Beispiel 5c:

### 5-[(3E/Z,1R,5R,6S,7R)-1-(4-Chlorphenylsulfonylaminomethyl)-6-[(1Z,3RS,4S)-2-brom-3-hydroxy-4-methyl-non-1-en-6-inyl]-7-(tetrahydropyran-2-yloxy)-bicyclo[3.3.0]oct-3- yliden]-pentansäuremethylester:

592 mg (788 µmol) der nach Beispiel 5d dargestellten Verbindung setzt man in Analogie zu Beispiel 1b um und isoliert nach Aufarbeitung und Reinigung 406 mg (538 µmol, 68%) der Titelverbindung als farbloses Öl.
IR (Film): 3600-3100, 3270, 3050, 2950, 2860, 1725, 1585, 1435, 1330, 1260, 1160, 1090, 1010, 870, 825, 735 und 700 cm⁻¹.

### Beispiel 5d:

### 5-[(3E/Z,1R,5R,6S,7R)-1-(4-Chlorphenylsulfonylaminomethyl)-6-[(1Z,4S)-2-brom-3-oxo-4 -methyl-non-1-en-6-inyl]-7-(tetrahydropyran-2-yloxy)-bicyclo[3.3.0]oct-3-yliden]-pentansäuremethylester:

Zu der Aufschlämmung von 63 mg Natriumhydrid-Dispersion (80%ig) in 8 ml wasserfreiem Dimethoxyethan tropft man bei 3°C unter einer Atmosphäre aus trockenem Argon die Lösung von 554 mg Dimethyl-(2-oxo-3S-methyl-oct-5-inyl)-phosphonat ( darstellbar nach z.B. Liebigs Ann. Chem. 1989, 1081-1083 ) in 5 ml wasserfreiem Dimethoxyethan und rührt 30 Minuten. Anschließend versetzt man innerhalb 20 Minuten portionsweise mit insgesamt 422 mg N-Bromsuccinimid, läßt weitere 30 Minuten reagieren, tropft zügig die Lösung von 538 mg (971 µmol) des nach Beispiel 1d dargestellten Rohaldehyds in 5 ml wasserfreiem Dimethoxyethan zu, läßt auf 23°C erwärmen und rührt 6 Stunden. Man gießt auf eine 10%ige Ammoniumchloridlösung und extrahiert mehrfach mit Diethylether. Die vereinigten organischen Extrakte wäscht man mit Wasser, trocknet über Magnesiumsulfat und reinigt den nach Lösungsmittelabzug erhaltenen Rückstand durch Chromatographie an ca. 50 ml feinem Kieselgel mit einem Gradientensystem aus aus n-Hexan und Ethylacetat. Isoliert werden 598 mg (794 µmol, 82%) der Titelverbindung als farbloses Öl.
IR (Film): 3270, 2940, 2870, 1735, 1680, 1610, 1585, 1450, 1435, 1335, 1250, 1160, 1070, 1030, 975, 870, 830 und 750 cm⁻¹.

### Beispiel 6:

### 5-[(3E/Z,1R,5R,6S,7R)-1-(4-Chlorphenylsulfonylaminomethyl)-6-[(3R,4S)-3-hydroxy-4-methyl-nona-1,6-diinyl]-7-hydroxy-bicyclo[3.3.0]oct-3-yliden]-pentansäure:

106 mg (180 µmol) der nach Beispiel 6a dargestellten Verbindung verseift man in Analogie zu Beispiel 1 und isoliert nach Aufarbeitung und Reinigung 88 mg (153 µmol, 85%) der Titelverbindung als farbloses Öl.
¹H-NMR (CDCl₃): δ= 1,03 (d,3H), 1,12 (t,3H), 1,41 (m,1H), 1,6-1,75 (m,2H), 1,88 (m,1H), 1,93-2,5 (m,16H), 2,77 (m,2H), 3,5-4,8 (s,1H), 4,02 (m,1H), 4,4 (m,1H), 5,22 (m,1H), 5,88 (m,1H), 7,49 (d,2H), 7,79 (d,2H).

### Beispiel 6a:

### 5-[(3E/Z,1R,5R,6S,7R)-1-(4-Chlorphenylsulfonylaminomethyl)-6-[(3R,4S)-3-hydroxy-4-methyl-nona-1,6-diinyl]-7-hydroxy-bicyclo[3.3.0]oct-3-yliden]-pentansäuremethylester:

188 mg (280 µmol) der nach Beispiel 5b dargestellten Verbindung B setzt man in Analogie zu Beispiel 5a um und isoliert nach Aufarbeitung und Reinigung 106 mg (180 µmol, 70%) der Titelverbindung als farbloses Öl.
¹H-NMR (CDCl₃): δ= 1,05 (d,3H), 1,11 (t,3H), 1,45 (m,1H), 1,6-1,75 (m,4H), 1,85-2,55 (m,16H), 2,82 (m,2H), 3,68 (s,3H), 4,08 (m,1H), 4,42 (m,1H), 5,24 (m,2H), 7,5 (d,2H), 7,79 (d,2H).

### Beispiel 7:

### 4-[(3E/Z,1R,5R,6S,7R)-1-(4-Chlorphenylsulfonylaminomethyl)-6-[(3S,4S)-3-hydroxy-4-methyl-nona-1,6-diinyl]-7-hydroxy-bicyclo[3.3.0]oct-3-yliden]-butansäure:

39 mg (68 µmol) der nach Beispiel 7a dargestellten Verbindung verseift man in Analogie zu Beispiel 1 und isoliert nach Aufarbeitung und Reinigung 34 mg (60 µmol, 89%) der Titelverbindung als farbloses Öl.
¹H-NMR (CD₃OD): δ= 1,03 (d,3H), 1,1 (t,3H), 1,78 (m,1H), 2,0-2,5 (m,15H), 2,73 (s,2H), 3,92 (m,1H), 4,3 (m,1H), 5,28 (m,1H), 7,58 (d,2H), 7,82 (d,2H).

### Beispiel 7a:

### 4-[(3E/Z,1R,5R,6S,7R)-1-(4-Chlorphenylsulfonylaminomethyl)-6-[(3S,4S)-3-hydroxy-4-methyl-nona-1,6-diinyl]-7-hydroxy-bicyclo[3.3.0]oct-3-yliden]-butansäuremethylester:

59 mg (90 µmol) der nach Beispiel 7b dargestellten Verbindung A setzt man in Analogie zu Beispiel 5a um und isoliert nach Aufarbeitung und Reinigung 39 mg (68 µmol, 76%) der Titelverbindung als farbloses Öl.
¹H-NMR (CDCl₃): δ= 1,05 (d,3H), 1,12 (t,3H), 1,49 (m,1H), 1,89 (m,1H), 2,05-2,55 (m,15H), 2,7-2,85 (m,4H), 3,66 (s,3H), 4,09 (m,1H), 4,37 (m,1H), 5,19 (m,1H), 5,36 (t,1H), 7,5 (d,2H), 7,82 (d,2H).

### Beispiel 7b:

### 4-[(3E/Z,1R,5R,6S,7R)-1-(4-Chlorphenylsulfonylaminomethyl)-6-[(1Z,3S,4S)-2-brom-3-hydroxy-4-methyl-non-1-en-6-inyl]-7-hydroxy-bicyclo[3.3.0]oct-3-yliden]-butansäuremethylester (A) und 5-[(3E/Z,1R,5R,6S,7R)-1-(4-Chlorphenylsulfonylaminomethyl)-6-[(1Z,3R,4S)-2-brom-3-hydroxy-4-methyl-non-1-en-6-inyl]-7-hydroxy-bicyclo[3.3.0]oct-3-yliden]-butansäuremethylester (B):

124 mg (187 µmol) der nach Beispiel 7c dargestellten Verbindungen setzt man in Analogie zu Beispiel 1a um und isoliert nach Aufarbeitung und Reinigung 56 mg (85 µmol, 45%) einer unpolaren Komponente, der man die Struktur B zuordnet, sowie 59 mg (90 µmol, 48%) einer polaren Komponente, der man die Struktur A zuordnet, jeweils als farbloses Öl.
IR (Film) von A und B: 3600-3100, 3270, 3050, 2960, 2860, 1725, 1590, 1430, 1330, 1260, 1160, 1090, 1010, 825, 735 und 700 cm⁻¹.

### Beispiel 7c:

### 4-[(3E/Z,1R,5R,6S,7R)-1-(4-Chlorphenylsulfonylaminomethyl)-6-[(1Z,3RS,4S)-2-brom-3-hydroxy-4-methyl-non-1-en-6-inyl]-7-(tetrahydropyran-2-yloxy)-bicyclo[3.3.0]oct-3-yliden]-butansäuremethylester:

177 mg (239 µmol) der nach Beispiel 7d dargestellten Verbindungen setzt man in Analogie zu Beispiel 1b um und isoliert nach Aufarbeitung und Reinigung 124 mg (187 µmol, 78%) der Titelverbindungen als farbloses Öl.
IR (Film): 3600-3100, 3270, 3050, 2960, 2860, 1725, 1585, 1430, 1330, 1260, 1160, 1090, 1010, 865, 825, 735 und 700 cm⁻¹.

### Beispiel 7d:

### 4-[(3E/Z,1R,5R,6S,7R)-1-(4-Chlorphenylsulfonylaminomethyl)-6-[(1Z,4S)-2-brom-3-oxo-4 -methyl-non-1-en-6-inyl]-7-(tetrahydropyran-2-yloxy)-bicyclo[3.3.0]oct-3-yliden]-butansäuremethylester:

191 mg (354 µmol) der nach Beispiel 3d dargestellten Verbindung setzt man in Analogie zu Beispiel 5d um und isoliert nach Aufarbeitung und Reinigung 177 mg (239 µmol, 68%) der Titelverbindung als farbloses Öl.
IR (Film): 3270, 3080, 2930, 2870, 2850, 1735, 1685, 1610, 1585, 1440, 1335, 1240, 1160, 1035, 970, 865, 825 und 750 cm⁻¹.

### Beispiel 8:

### 4-[(3E/Z,1R,5R,6S,7R)-1-(4-Chlorphenylsulfonylaminomethyl)-6-[(3R,4S)-3-hydroxy-4-methyl-nona-1,6-diinyl]-7-hydroxy-bicyclo[3.3.0]oct-3-yliden]-butansäure:

35 mg (61 µmol) der nach Beispiel 8a dargestellten Verbindung verseift man in Analogie zu Beispiel 1 und isoliert nach Aufarbeitung und Reinigung 29 mg (52 µmol, 76%) der Titelverbindung als farbloses Öl.
¹H-NMR (CD₃OD): δ= 1,03 (d,3H), 1,1 (t,3H), 1,76 (m,1H), 2,0-2,5 (m,15H), 2,74 (s,2H), 3,92 (m,1H), 4,3 (m,1H), 5,28 (m,1H), 7,57 (d,2H), 7,82 (d,2H).

### Beispiel 8a:

### 4-[(3E/Z,1R,5R,6S,7R)-1-(4-Chlorphenylsulfonylaminomethyl)-6-[(3R,4S)-3-hydroxy-4-methyl-nona-1,6-diinyl]-7-hydroxy-bicyclo[3.3.0]oct-3-yliden]butansäuremethylester:

56 mg (85 µmol) der nach Beispiel 7b dargestellten Verbindung B setzt man in Analogie zu Beispiel 5a um und isoliert nach Aufarbeitung und Reinigung 35 mg (61 µmol, 71%) der Titelverbindung als farbloses Öl.
¹H-NMR (CDCl₃): δ= 1,05 (d,3H), 1,12 (t,3H), 1,49 (m,1H), 1,90 (m,1H), 2,05-2,55 (m,15H), 2,65-2,85 (m,4H), 3,66 (s,3H), 4,09 (m,1H), 4,42 (m,1H), 5,19 (m,1H), 5,32 (t,1H), 7,5 (d,2H), 7,82 (d,2H).

### Beispiel 9:

### 5-[(3E,1R,5R,6R,7R)-1-[5-Phenylsulfonylamino-1-pentinyl]-6-[(1E,3S,4RS)-3-hydroxy-4-methyl-oct-1-en-6-inyl]-7-hydroxy-bicyclo[3.3.0]oct-3-yliden]-pentansäure:

16,7 mg (28 µmol) der nach Beispiel 9a dargestellten Verbindung verseift man in Analogie zu Beispiel 1 und isoliert nach Aufarbeitung und Reinigung 16,5 mg (28 µmol, 100%) der Titelverbindung als farbloses Öl.
¹H-NMR (CDCl₃): δ= 0,92 und 1,03 (jeweils d, insgesamt 3H), 1,45-1,85 (m,10H), 1,95-2,55 (m,14H), 3,06 (m,2H), 3,5-4,5 (m,5H), 5,05 (m,1H), 5,22 (m,1H), 5,53 (m,2H), 7,55 (m,3H), 7,87 (d,2H).

### Beispiel 9a:

### 5-[(3E,1R,5R,6R,7R)-1-[5-Phenylsulfonylamino-1-pentinyl]-6-[(1E,3S,4RS)-3-hydroxy-4-methyl-oct-1-en-6-inyl]-7-hydroxy-bicyclo[3.3.0]oct-3-yliden]-pentansäuremethylester:

22 mg (29 µmol) der nach Beispiel 9b dargestellten Verbindung setzt man in Analogie zu Beispiel 1a um und isoliert nach Aufarbeitung und Reinigung 16,7 mg (28 µmol, 97%) der Titelverbindung als farbloses Öl.
IR (Film): 3260, 3050, 2940, 2850, 1725, 1435, 1330, 1160, 1125, 1075, 1020, 970, 900, 810, 735 und 695 cm⁻¹.

### Beispiel 9b:

### 5-[(3E,1R,5R,6R,7R)-1-[5-Phenylsulfonylamino-1-pentinyl]-6-[(1E,3S,4RS)-3-hydroxy-4-methyl-oct-1-en-6-inyl]-7-(tetrahydropyran-2-yloxy)-bicyclo[3.3.0]oct-3-yliden]-pentansäuremethylester:

30,7 mg (49 µmol) der nach Beispiel 9c dargestellten Verbindung setzt man in Analogie zu Beispiel 1h unter Verwendung von Benzolsulfonsäurechlorid um und isoliert nach Aufarbeitung und Reinigung 22 mg (29 µmol, 59%) der Titelverbindung als farbloses Öl.
IR (Film): 3270, 3050, 2940, 2860, 1730, 1440, 1330, 1160, 1130, 1075, 1020, 970, 900, 865, 810, 735 und 690 cm⁻¹.

### Beispiel 9c:

### 5-[(3E,1R,5R,6R,7R)-1-[5-Amino-1-pentinyl]-6-[(1E,3S,4RS)-3-hydroxy-4-methyl-oct-1-en-6-inyl]-7-(tetrahydropyran-2-yloxy)-bicyclo[3.3.0]oct-3-yliden]-pentansäuremethylester:

116 mg (178 µmol) 5-[(3E,1R,5R,6R,7R)-1-[5-Azido-1-pentinyl]-6-[(1E,3S,4RS)-3-hydroxy-4-methyl-oct-1-en-6-inyl]-7-(tetrahydropyran-2-yloxy)-bicyclo[3.3.0]oct-3-yliden] -pentansäuremethylester, den man wie in DE 3725031 beschrieben herstellt, löst man in 1,56 ml wasseifreiem Tetrahydrofuran, versetzt mit 56 mg Triphenylphosphin und rührt 16 Stunden bei 23°C unter einer Atmosphäre aus trockenem Argon. Anschließend versetzt man mit 0,31 ml Wasser und erhitzt 1 Stunde zum Rückfluß. Man engt ein und reinigt durch Chromatographie an 5 analytischen Dünnschichtplatten. Als Laufmittel dient ein Gemisch aus n-Hexan und Aceton, als Elutionsmittel ein Gemisch aus Aceton, Chloroform und Ethylacetat. Isoliert werden 88 mg (141 mg, 79%) der Titelverbindung als farbloses Öl.
IR (Film): 3600-3100, 2930, 2830, 1735, 1435, 1200, 1130, 1075, 1020, 970, 865, 815 und 735 cm⁻¹.

### Beispiel 10:

### 5-[(3E,1R,5R,6R,7R)-1-[5-(4-Methylphenylsulfonylamino)-1-pentinyl]-6-[(1E,3S,4RS)-3 hydroxy-4-methyl-oct-1-en-6-inyl]-7-hydroxy-bicyclo[3.3.0]oct-3-yliden]-pentansäure:

19,5 mg (32 µmol) der nach Beispiel 10a dargestellten Verbindung verseift man in Analogie zu Beispiel 1 und isoliert nach Aufarbeitung und Reinigung 18,7 mg (31 µmol, 98%) der Titelverbindung als farbloses Öl.
¹H-NMR (CDCl₃): δ= 0,92 und 1,03 (jeweils d, insgesamt 3H), 1,45-1,85 (m,10H), 1,9-2,55 (m, 14H), 3,02 (m,2H), 3,5-4,6 (s,3H), 3,92 (m,1H), 4,03 (m,1H), 5,01 (m,1H), 5,22 (m,1H), 5,53 (m,2H), 7,32 (d,2H), 7,75 (d,2H).

### Beispiel 10a:

### 5-[(3E,1R,5R,6R,7R)-1-[5-(4-Methylphenylsulfonylamino)-1-pentinyl]-6-[(1E,3S,4RS)-3-hydroxy-4-methyl-oct-1-en-6-inyl]-7-hydroxy-bicyclo[3.3.0]oct-3-yliden]-pentansäuremethylester:

29 mg (37 µmol) der nach Beispiel 10b dargestellten Verbindung setzt man in Analogie zu Beispiel 1a um und isoliert nach Aufarbeitung und Reinigung 19,5 mg (32 µmol, 86%) der Titelverbindung als farbloses Öl.
IR (Film): 3600-3100, 3280, 3050, 2940, 2870, 1730, 1600, 1435, 1325, 1265, 1160, 1090, 970, 815, 735, 700 und 660 cm⁻¹.

### Beispiel 10b:

### 5-[(3E,1R,5R,6R,7R)-1-[5-(4-Methylphenylsulfonylamino)-1-pentinyl]-6-[(1E,3S,4RS)-3-hydroxy-4-methyl-oct-1-en-6-inyl]-7-(tetrahydropyran-2-yloxy)-bicyclo[3.3.0]oct-3-yliden] -pentansäuremethylester:

24 mg (38 µmol) der nach Beispiel 9c dargestellten Verbindung setzt man in Analogie zu Beispiel 1h unter Verwendung von 4-Methylbenzolsulfonsäurechlorid um und isoliert nach Aufarbeitung und Reinigung 29 mg (37 µmol, 97%) der Titelverbindung als farbloses Öl.
IR (Film): 3600-3100, 3270, 3050, 2940, 2860, 1730, 1600, 1435, 1325, 1260, 1160, 1090, 970, 865, 815, 735, 700 und 660 cm⁻¹.

### Beispiel 11:

### 5-[(3E,1R,5R,6R,7R)-1-[5-(4-Chlorphenylsulfonylamino)-1-pentinyl]-6-[(1E,3S,4RS)-3-hydroxy-4-methyl-oct-1-en-6-inyl]-7-hydroxy-bicyclo[3.3.0]oct-3-yliden]-pentansäure:

18,7 mg (30 µmol) der nach Beispiel 11a dargestellten Verbindung verseift man in Analogie zu Beispiel 1 und isoliert nach Aufarbeitung und Reinigung 16,4 mg (27 µmol, 89%) der Titelverbindung als farbloses Öl.
¹H-NMR (CDCl₃): δ= 0,92 und 1,03 (jeweils d, insgesamt 3H), 1,45-1,85 (m,10H), 1,95-2,55 (m,14H), 3,07 (m,2H), 3,5-4,5 (m,5H), 5,12 (m,1H), 5,22 (m,1H), 5,53 (m,2H), 7,5 (d,2H), 7,81 (d,2H).

### Beispiel 11a:

### 5-[(3E,1R,5R,6R,7R)-1-[5-(4-Chlorphenylsulfonylamino)-1-pentinyl]-6-[(1E,3S,4RS)-3-hydroxy-4-methyl-oct-1-en-6-inyl]-7-hydroxy-bicyclo[3.3.0]oct-3-yliden]-pentansäuremethylester:

25 mg (31 µmol) der nach Beispiel 11b dargestellten Verbindung setzt man in Analogie zu Beispiel 1a um und isoliert nach Aufarbeitung und Reinigung 18,7 mg (30 µmol, 97%) der Titelverbindung als farbloses Öl.
IR (Film): 3270, 2940, 2860, 1730, 1585, 1435, 1330, 1160, 1120, 1080, 1015, 970, 815, 750 und 735 cm⁻¹.

### Beispiel 11b:

### 5-[(3E,1R,5R,6R,7R)-1-[5-(4-Chlorphenylsulfonylamino)-1-pentinyl]-6-[(1E,3S,4RS)-3-hydroxy-4-methyl-oct-1-en-6-inyl]-7-(tetrahydropyran-2-yloxy)-bicyclo[3.3.0]oct-3-yliden] -pentansäuremethylester:

30,6 mg (49 µmol) der nach Beispiel 9c dargestellten Verbindung setzt man in Analogie zu Beispiel 1h um und isoliert nach Aufarbeitung und Reinigung 25 mg (31 µmol, 63%) der Titelverbindung als farbloses Öl.
IR (Film): 3270, 2940, 2860, 1735, 1585, 1435, 1335, 1160, 1125, 1080, 1020, 970, 865, 815, 750 und 735 cm⁻¹.

### Beispiel 12:

### 5-[(3E,1R,5R,6R,7R)-1-[5-(4-Fluorphenylsulfonylamino)-1-pentinyl]-6-[(1E,3S,4RS)-3-hydroxy-4-methyl-oct-1-en-6-inyl]-7-hydroxy-bicyclo[3.3.0]oct-3-yliden]-pentansäure:

16,5 mg (27 µmol) der nach Beispiel 12a dargestellten Verbindung verseift man in Analogie zu Beispiel 1 und isoliert nach Aufarbeitung und Reinigung 15 mg (25 µmol, 93%) der Titelverbindung als farbloses Öl.
¹H-NMR (CDCl₃): δ= 0,92 und 1,03 (jeweils d, insgesamt 3H), 1,45-1,85 (m,10H), 1,95-2,55 (m,14H), 3,05 (m,2H), 3,5-4,3 (m,5H), 5,12 (m,1H), 5,22 (m,1H), 5,52 (m,2H), 7,20 (m,2H), 7,88 (m,2H).

### Beispiel 12a:

### 5-[(3E,1R,5R,6R,7R)-1-[5-(4-Fluorphenylsulfonylamino)-1-pentinyl]-6[(1E,3S,4RS)-3-hydroxy-4-methyl-oct-1-en-6-inyl]-7-hydroxy-bicyclo[3.3.0]oct-3-yliden]-pentansäuremethylester:

21 mg (27 µmol) der nach Beispiel 12b dargestellten Verbindung setzt man in Analogie zu Beispiel 1a um und isoliert nach Aufarbeitung und Reinigung 16,5 mg (27 µmol, 100%) der Titelverbindung als farbloses Öl.

### Beispiel 12b:

### 5-[(3E,1R,5R,6R,7R)-1-[5-(4-Fluorphenylsulfonylamino)-1-pentinyl]-6-[(1E,3S,4RS)-3-hydroxy-4-methyl-oct-1-en-6-inyl]-7-(tetrahydropyran-2-yloxy)-bicyclo[3.3.0]oct-3-yliden] -pentansäuremethylester:

31 mg (50 µmol) der nach Beispiel 9c dargestellten Verbindung setzt man in Analogie zu Beispiel 1h unter Verwendung von 4-Fluorbenzolsulfonsäurechlorid um und isoliert nach Aufarbeitung und Reinigung 21 mg (27 µmol, 54%) der Titelverbindung als farbloses Öl.

### Beispiel 13:

### 5-[(3E,1S,5R,6R,7R)-1-[5-(4-Methylphenylsulfonylamino)-1-pentyl]-6-[(1E,3S,4RS)-3-hydroxy-4-methyl-oct-1-en-6-inyl]-7-hydroxy-bicyclo[3.3.0]oct-3-yliden]-pentansäure:

15 mg (24 µmol) der nach Beispiel 13a dargestellten Verbindung verseift man in Analogie zu Beispiel 1 und isoliert nach Aufarbeitung und Reinigung 12,3 mg (21 µmol, 87%) der Titelverbindung als farbloses Öl.
¹H-NMR (CD₃OD): δ= 0,92 und 1,03 (jeweils d, insgesamt 3H), 1,1-1,5 (m,11H), 1,55-2,5 (m, 17H), 2,43 (s,3H), 3,07 (t,2H), 3,72 (m,1H), 3,92 (m,1H), 5,22 (m,1H), 5,5 (m,2H), 7,38 (d,2H), 7,83 (d,2H).

### Beispiel 13a:

### 5-[(3E,1S,5R,6R,7R)-1-[5-(4-Methylphenylsulfonylamino)-1-pentyl]-6-[(1E,3S,4RS)-3-hydroxy-4-methyl-oct-1-en-6-inyl]-7-hydroxy-bicyclo[3.3.0]oct-3-yliden]-pentansäuremethylester:

19,5 mg (25 µmol) der nach Beispiel 13b dargestellten Verbindung setzt man in Analogie zu Beispiel 1a um und isoliert nach Aufarbeitung und Reinigung 15 mg (24 µmol, 98%) der Titelverbindung als farbloses Öl.
IR (Film): 3600-3100, 3050, 2920, 2850, 1730, 1670, 1600, 1535, 1440, 1340, 1260, 1160, 1090, 1010, 970, 890, 815, 735, 700 und 660 cm⁻¹.

### Beispiel 13b:

### 5-[(3E,1S,5R,6R,7R)-1-[5-(4-Methylphenylsulfonylamino)-1-pentyl]-6-[(1E,3S,4RS)-3-(tetrahydropyran-2-yloxy)-4-methyl-oct-1-en-6-inyl]-7-(tetrahydropyran-2-yloxy)-bicyclo-[3.3.0]oct-3-yliden]-pentansäuremethylester:

23 mg (37 µmol) der nach Beispiel 13c dargestellten Verbindung setzt man in Analogie zu Beispiel 1h unter Verwendung von 4-Methylbenzolsulfonsäurechlorid um und isoliert nach Aufarbeitung und Reinigung 19,5 mg (25 µmol, 68%) der Titelverbindung als farbloses Öl.
IR (Film): 3340, 3050, 2930, 2850, 1735, 1665, 1540, 1450, 1340, 1255, 1200, 1160, 1125, 1075, 1020, 975, 865, 815, 735, 700 und 665 cm⁻¹.

### Beispiel 13c:

### 5-[(3E,1S,5R,6R,7R)-1-[5-Amino-1-pentyl]-6-[(1E,3S,4RS)-3-(tetrahydropyran-2-yloxy)-4-methyl-oct-1-en-6-inyl]-7-(tetrahydropyran-2-yloxy)-bicyclo[3.3.0]oct-3-yliden]-pentansäuremethylester:

26,4 mg (40 µmol) 5-[(3E,1S,5R,6R,7R)-1-[5-Azido-1-pentyl]-6-[(1E,3S,4RS)-3-(tetrahydropyran-2-yloxy)-4-methyl-oct-1-en-6-inyl]-7-(tetrahydropyran-2-yloxy)-bicyclo[3.3.0] oct-3-yliden]-pentansäuremethylester, den man wie in DE 3742745 A1 und US 4,971,987 beschrieben, herstellt, setzt man in Analogie zu Beispiel 9c um und isoliert nach Aufarbeitung und Reinigung 23 mg (37 µmol, 91%) der Titelverbindung als farbloses Öl.
IR (Film): 3340, 2930, 2850, 1735, 1630, 1570, 1435, 1255, 1200, 1130, 1075, 1030, 1020, 975, 905, 865, 815, 735 und 700 cm⁻¹.

### Beispiel 14:

### 5-[(3E/Z,1R,5R,6R,7R)-1-(4-Chlorphenylsulfonylaminomethyl)-6-[(1E,3R oder 3S)-3-hydroxy-oct-1-enyl]-7-hydroxy-bicyclo[3.3.0]oct-3-yliden]-pentansäure:

11 mg (19 µmol) der nach Beispiel 14a dargestellten Verbindung verseift man in Analogie zu Beispiel 1 und isoliert nach Aufarbeitung und Reinigung 10,3 mg (18 µmol, 95%) der Titelverbindung als farbloses Öl.
¹H-NMR (CDCl₃): δ= 0,89 (t,3H), 1,20-1,60 (m,10H), 1,70 (m,3H), 1,90-2,1 (m,4H), 2,2-2,4 (m,5H), 2,75 (s,2H), 3,8 (m,1H), 4,1 (m,1H), 4,2-4,5 (breit,2H), 5,2 (s,1H), 5,6 (s,2H), 6,05 (s, breit,1H), 7,5 (d,2H), 7,8 (d,2H).

### Beispiel 14a:

### 5-(3E/Z,1R,5R,6R,7R)-1-(4-Chlorphenylsulfonylaminomethyl)-6[(1E,3R oder 3S)-3-hydroxy-oct-1-enyl]-7-hydroxy-bicyclo[3.3.0]oct-3-yliden]-pentansäuremethylester:

17,4 mg (27 µmol) der nach Beispiel 14b dargestellten Verbindung A setzt man in Analogie zu Beispiel 1a um und isoliert nach Aufarbeitung und Reinigung 11 mg (19 µmol, 70%) der Titelverbindung als farbloses Öl.
IR (Film): 3600-3100, 3053, 1725, 1595, 1500, 1330, 1260, 1095, 865, 740, 735 cm⁻¹.

### Beispiel 14b:

### 5-[(3E/Z,1R,5R,6R,7R)-1-(4-Chlorphenylsulfonylaminomethyl)-6-[(1E,3R oder 3S)-3-hydroxy-oct-1-enyl]-7-(tetrahydropyran-2-yloxy)-bicyclo[3.3.0]oct-3-yliden]-pentansäuremethylester (A) und 5-[(3E/Z,1R,5R,6R,7R)-1-(4-Chlorphenylsulfonylaminomethyl)-6-[(1E,3S oder 3R)-3-hydroxy-oct-1-enyl]-7-(tetrahydropyran-2-yloxy)-bicyclo[3.3.0]oct-3-yliden]-pentansäuremethylester (B):

55 mg (84 µmol) der nach Beispiel 14c dargestellten Verbindung setzt man in Analogie zu Beispiel 1b um und isoliert nach Aufarbeitung und Reinigung 20,5 mg (31 µmol, 37%) einer unpolaren Komponente, der man die Struktur B zuordnen sowie 17,4 mg (27 µmol, 32%) einer polaren Komponente, der man die Stur A zuordnet, jeweils als farbloses Öl.
IR (Film) von A und B: 3600-3100, 3260, 3000, 2970, 1720, 1595, 1420, 1330, 1250, 1165, 1080, 830, 735 und 700 cm⁻¹.

### Beispiel 14c:

### 5-[(3E/Z,1R,5R,6R,7R)-1-(4-Chlorphenylsulfonylaminomethyl)-6-[(1E)-3-oxo-oct-1-enyl]-7-(tetrahydropyran-2-yloxy)-bicyclo[3.3.0]oct-3-yliden]-pentansäuremethylester:

Zu der Aufschlämmung von 7,4 mg Natriumhydrid-Dispersion (80%ig) in 1,8 ml wasserfreiem Tetrahydrofuran gibt man unter Argon bei Raumtemperatur 56,6 mg Dimethyl-(2-oxo-heptyl)-phosphonat. Nach Beendigung der Gasentwicklung kühlt man auf -30°C und versetzt mit 113,9 mg des nach Beispiel 1d dargestellten Rohaldehyds gelöst in 0,7 ml Tetrahydrofuran. Anschließend läßt man bei einer Temperatur zwischen -25°C und 2°C 3,5 Stunden reagieren. Zur Aufarbeitung gibt man 0,11 ml Essigsäure zu, läßt auf Raumtemperatur kommen, verdünnt mit Diethylether, wacht einmal mit 10%iger Ammoniumchloridlösung und dreimal mit Wasser. Nach Trocknung der vereinigten organischen Phasen über Magnesiumsulfat und Chromatographie an Kieselgel mit Chloroform/Diethylether im Verhältnis 8/2 isoliert man 55 mg (84 µmol, 41%) der Titelverbindung.
IR (Film): 3090, 3065, 3000, 2985, 1725, 1700, 1590, 1500, 1360, 1300, 1240, 1190, 1010, 835, 780 und 735 cm⁻¹.

### Beispiel 15:

### 5-[(3E/Z,1R,5R,6R,7R)-1-(4-Chlorphenylsulfonylaminomethyl)-6-[(1E,3S oder 3R)-3-hydroxy-oct-1-enyl]-7-hydroxy-bicyclo[3.3.0]oct-3-yliden]pentansäure

22,1 mg (38 µmol) der nach Beispiel 15a dargestellten Verbindung verseift man in Analogie zu Beispiel 1 und isoliert nach Aufarbeitung und Reinigung 18,1 mg (32 µmol, 84%) der Titelverbindung als farbloses Öl.
¹H-NMR (CDCl₃): 0,9 (t,3H), 1.20-1,85 (m,13H), 1,85-2,15 (m,5H), 2,20-2,40 (m,4H), 2,75 (d,2H), 3,80 (q,1H), 4,05 (m,1H), 4,20-4,60 (breit,3H), 5,20 (s,1H), 5,50 (m,2H), 6,20 (s,1H), 7,49 (d,2H), 7,80 (d,2H).

### Beispiel 15a:

### 5-[(3E/Z,1R,5R,6R,7R)-1-(4-Chlorphenylsulfonylaminomethyl)-6-[(1E,3S oder 3R)-3-hydroxy-oct-1-enyl]-7-hydroxy-bicyclo[3.3.0]oct-3-yliden]-pentansäuremethylester:

20,5 mg (31 µmol) der nach Beispiel 14b dargestellten Verbindung B setzt man in Analogie zu Beispiel 1a um und isoliert nach Aufarbeitung 17 mg (33 µmol, 96%) der Titelverbindung als farbloses Öl.
IR (Film): 3600-3100, 3055, 1725, 1590, 1500, 1325, 1260, 1095, 860 und 735 cm⁻¹.

### Beispiel 16:

### 5-[(3E/Z,1R,5R,6R,7R)-1-(4-Chlorphenylsulfonylaminomethyl)-6-[(3R oder 3S)-3-hydroxy-oct-1-inyl]-7-hydroxy-bicyclo[3.3.0]oct-3-yliden]-pentansäure

9,2 mg (16 µmol) der nach Beispiel 16a dargestellten Verbindung verseift man in Analogie zu Beispiel 1 und isoliert nach Aufarbeitung und Reinigung 7,4 mg (14 µmol, 88%) der Titelverbindung als farbloses Öl.
¹H-NMR (CDCl₃): δ= 0,90 (t,3H), 1,20-1,45 (m,7H), 1,60-1,80 (m,4H), 1,95-2,50 (m,11H), 2,80 (m,2H), 4,0 (breit,3H), 4,45 (t,1H), 5,20 (s,1H), 5,98 (s,1H), 7,50 (d,2H), 7,70 (d,2H).

### Beispiel 16a:

### 5-[(3E/Z,1R,5R,6R,7R)-1-(4-Chlorphenylsulfonylaminomethyl)-6-[(3R oder 3S)-3-hydroxy-oct-1-inyl]-7-hydroxy-bicyclo[3.3.0]oct-3-yliden]-pentansäuremethylester:

13,7 mg (21 µmol) der nach 16b dargestellten Verbindung setzt man in Analogie zu Beispiel 5a um und isoliert nach Aufarbeitung und Reinigung 9,2 mg (16 µmol, 76%) der Titelverbindung als farbloses Öl.
IR (Film): 3600-3120, 3010, 3000, 2970, 1725, 1590, 1495, 1380, 1320, 1250, 1200, 1160, 1080, 1000, 840, 735 und 700 cm⁻¹.

### Beispiel 16b:

### 5-[(3E/Z,1R,5R,6R,7R)-1-(4-Chlorphenylsulfonylaminomethyl)-6-[(1Z,3R oder 3S)-2-brom-3-hydroxy-oct-1-enyl]-7-hydroxy-bicyclo[3.3.0]oct-3-yliden]-pentansäuremethylester:

20,5 mg (28 µmol) der nach 16c dargestellten Verbindung A setzt man in Analogie zu Beispiel 1a um und isoliert nach Aufarbeitung und Reinigung 13,7 mg (21 µmol, 75%) der Titelverbindung als farbloses Öl.
IR (Film): 3650-3100, 3040, 3010, 3000, 2960, 1725, 1580, 1500, 1310, 1290, 1220, 1180, 1050, 1000, 870, 790 und 740 cm⁻¹.

### Beispiel 16c:

### 5-[(3E/Z,1R,5R,6R,7R)-1-(4-Chlorphenylsulfonylaminomethyl)-6-[(1Z,3R oder 3S)-2-brom-3-hydroxy-oct-1-enyl]-7-(tetrahydropyran-2-yloxy)-bicyclo[3.3.0]oct-3-yliden]-pentansäuremethylester (A) und 5-[(3E/Z,1R,5R,6R,7R)-1-(4-Chlorphenylsulfonylaminomethyl)-6-[(1Z,3S oder 3R)-2-brom-3-hydroxy-oct-1-enyl]-7-(tetrahydropyran-2-yloxy)-bicyclo[3.3.0]oct-3-yliden]-pentansäuremethylester (B):

104 mg (142 µmol) der nach Beispiel 16d dargestellten Verbindung setzt man in Analogie zu Beispiel 1b um und isoliert nach Aufarbeitung und Reinigung 34,3 mg (46 µmol, 32%) einer unpolaren Komponente, der man die Struktur B zuordnet, sowie 20,5 mg (28 µmol, 20%) einer polaren Komponente, der man die Struktur A zuordnet, jeweils als farbloses Öl.
IR (Film) von A und B: 3600-3100, 3020, 3010, 3000, 2890, 1725, 1590, 1505, 1310, 1280, 1220, 1175, 1050, 1000, 870, 790 und 735 cm⁻¹

### Beispiel 16d:

### 5-[(3E/Z,1R,5R,6R,7R)-1-(4-Chlorphenylsulfonylaminomethyl)-6-[(1Z)-2-brom-3-oxo-oct-1-enyl]-7-(tetrahydropyran-2-yloxy)-bicyclo[3.3.0]oct-3-yliden]-pentansäuremethylester:

Zu der Aufschlämmung von 8,8 mg Natriumhydrid-Dispersion (80%) in 1,9 ml wasserfreiem Tetrahydrofuran tropft man bei Raumtemperatur unter einer Atmosphäre aus trockenem Argon die Lösung von 67,7 mg Dimethyl-(2-oxo-heptyl)-phosphonat in 0,6 ml wasserfreiem Tetrahydrofuran und rührt 10 Minuten. Anschließend wird auf 0°C abgekühlt und mit 57,8 mg N-Bromsuccinimid versetzt. Man läßt eine Stunde reagieren und tropft dann 112,5 mg (203 µmol) des nach Beispiel 1d dargestellten Rohaldehyds gelöst in 0,7 ml Tetrahydrofuran hinzu. Man läßt auf 23°C erwärmen, rührt 5,5 Stunden, gießt dann auf 10%ige Ammoniumchloridlösung und extrahiert mehrfach mit Diethylether. Die vereinigten organischen Phasen wäscht man mit Wasser, trocknet über Magnesiumsulfat und reinigt den nach Lösungsmittelabzug erhaltenen Rückstand durch präparative Plattenchromatographie mit n-Hexan/Essigester im Verhältnis 7/3 als Elutionsmittel. Isoliert werden 104 mg (143 µmol, 70%) der Titelverbindung als farbloses Öl.
IR (Film): 3280, 3010,2940, 1725, 1680, 1610, 1595, 1465, 1330, 1250, 1160, 1000, 975, 840, 785 und 750 cm⁻¹.

### Beispiel 17:

### 5-[(3E/Z,1R,5R,6R,7R)-1-(4-Chlorphenylsulfonylaminomethyl)-6-[(3S oder 3R)-3-hydroxy-oct-1-inyl]-7-hydroxy-bicyclo[3.3.0]oct-3-yliden]-pentansäure:

15,4 mg (27 µmol) der nach Beispiel 17a dargestellten Verbindung verseift man in Analogie zu Beispiel 1 und isoliert nach Aufarbeitung und Reinigung 11,7 mg (21 µmol, 78%) der Titelverbindung als farbloses Öl.
¹H-NMR (CDCl₃): δ= 0,90 (t,3H), 1,20-1,50 (m,7H), 1,60-1,75 (m,4H), 1,95-2,45 (m, 11H), 2,75 (s,2H), 4,0 (m,1H), 4,10-4,35 (breit,3H), 4,37 (t,1H), 5,25 (m,1H), 6,05 (breit,1H), 7,50 (d,2H), 7,80 (d,2H).

### Beispiel 17a:

### 5-[(3E/Z,1R,5R,6R,7R)-1-(4-Chlorphenylsulfonylaminomethyl)-6-[(3S oder 3R)-3-hydroxy-oct-1-inyl]-7-hydroxy-bicyclo[3.3.0]oct-3-yliden]-pentansäuremethylester:

29 mg (45 µmol) der nach 17b dargestellten Verbindung setzt man in Analogie zu Beispiel 5a um und isoliert nach Aufarbeitung und Reinigung 15,4 mg (27 µmol, 60%) der Titelverbindung als farbloses Öl.
IR (Film): 3600-3100, 3010, 3000, 2980, 1725, 1585, 1495, 1380, 1320, 1250, 1200, 1160, 1080, 1000, 840, 735 und 700 cm⁻¹.

### Beispiel 17b:

### 5-[(3E/Z,1R,5R,6R,7R)-1-(4-Chlorphenylsulfonylaminomethyl)-6-[(1Z,3S oder 3R)-2-brom-3-hydroxy-oct-1-enyl]-7-hydroxy-bicyclo[3.3.0]oct-3-yliden]-pentansäuremethylester:

34,3 mg (47 µmol) der nach 16c dargestellten Verbindung B setzt man in Analogie zu Beispiel 1a um und isoliert nach Aufarbeitung und Reinigung 29 mg (45 µmol, 96%) der Titelverbindung als farbloses Öl.
IR (Film): 3600-3100, 3040, 3010, 3000, 2980, 1725, 1590, 1500, 1310, 1290, 1240, 1180, 1050, 1010, 870, 790 und 740 cm⁻¹.

### Beispiel 18:

### 5-[(3E/Z,1R,5R,6R,7R)-1-(4-Chlorphenylsulfonylaminomethyl)-6-[(1E,3R oder 3S)-3-hydroxy-3-cyclohexyl-prop-1-enyl]-7-hydroxy-bicyclo[3.3.0]oct-3-yliden]-pentansäure:

12,4 mg (21 µmol) der nach Beispiel 18a dargestellten Verbindung verseift man in Analogie zu Beispiel 1 und isoliert nach Aufarbeitung und Reinigung 10,3 mg (18 µmol, 85%) der Titelverbindung als farbloses Öl.
¹H-NMR (CDCl₃): δ= 0,9-1,05 (m,2H), 1,10-1,30 (m,3H), 1,30-1,50 (m,3H), 1,60-1,85 (m,8H), 1,90-2,1 (m,5H), 2,20-2,40 (m,5H), 2,75 (s,2H), 3,80 (s,2H), 4,10-4,50 (breit,3H), 5,20 (s,1H), 5,55 (s,2H), 6,0 (breit,1H), 7,50 (d,2H), 7,80 (d,2H).

### Beispiel 18a:

### 5-[(3E/Z,1R,5R,6R,7R)-1-(4-Chlorphenylsulfonylaminomethyl)-6-[(1E,3R oder 3S)-3-hydroxy-3-cyclohexyl-prop-1-enyl]-7-hydroxy-bicyclo[3.3.0]oct-3-yliden]-pentansäuremethylester:

19,5 mg (29 µmol) der nach 18b dargestellten Verbindung A setzt man in Analogie zu Beispiel 5a um und isoliert nach Aufarbeitung und Reinigung 12,4 mg (21 µmol, 72%) der Titelverbindung als farbloses Öl.
IR (Film): 3600-3110, 3090, 3010, 2990, 2940, 1725, 1610, 1585, 1450, 1335, 1250, 1160, 1070, 1020, 975, 830 und 750 cm⁻¹.

### Beispiel 18b:

### 5-[(3E/Z,1R,5R,6R,7R)-1-(4-Chlorphenylsulfonylaminomethyl)-6-[(1E,3R oder 3S)-3-hydroxy-3-cyclohexyl-prop-1-enyl]-7-(terahydropyran-2-yloxy)-bicyclo[3.3.0]oct-3-yliden] -pentansäuremethylester (A) und 5-[(3E/Z,1R,5R,6R,7R)-1-(4-Chlorphenylsulfonylaminomethyl)-6-[(1E,3S oder 3R)-3-hydroxy-3-cyclohexyl-prop-1-enyl]-7-(terahydropyran-2-yloxy)-bicyclo[3.3.0]oct-3-yliden]-pentansäuremethylester (B):

72 mg (108 µmol) der nach Beispiel 18c dargestellten Verbindung setzt man in Analogie zu Beispiel 1b um und isoliert nach Aufarbeitung und Reinigung 42,3 mg (64 µmol, 59%) einer unpolaren Komponente, der man die Struktur B zuordnet, sowie 19,5 mg (29 µmol, 27%) einer polaren Komponente, der man die Struktur A zuordnet, jeweils als farbloses Öl.
IR (Film) von A und B: 3600-3100, 3020, 3000, 2980, 2970, 1735, 1600, 1460, 1435, 1360, 1310, 1280, 1220, 1110, 1020, 975, 870, 750 und 735 cm⁻¹.

### Beispiel 18c:

### 5-[(3E/Z,1R,5R,6R,7R)-1-(4-Chlorphenylsulfonylaminomethyl)-6-[(1E)-3-oxo-3-cyclohexyl-prop-1-enyl]-7-(terahydropyran-2-yloxy)-bicyclo[3.3.0]oct-3-yliden]-pentan-säuremethylester:

Zu der Aufschlämmung von 7,5 mg Natriumhydrid-Dispersion (80%) in 1,9 ml wasserfreiem Tetrahydrofuran gibt man unter Argon bei Raumtemperatur 60,4 mg Dimethyl-(2-oxo-2-cyclohexylethyl)-phosphonat. Nach Beendigung der Gasentwicklung kühlt man auf -30°C und versetzt mit 115,3 mg des nach Beispiel 1d dargestellten Rohaldehyds gelöst in 0,7 ml wasserfreiem Tetrahydrofuran. Anschließend läßt man bei einer Temperatur zwischen -25°C und 2°C 3,5 Stunden reagieren. Zur Aufarbeitung gibt man 0,11 ml Essigsäure zu, läßt auf Raumtemperatur komme verdünnt mit Diethylether, wäscht einmal mit 10% Ammoniumchloridlösung und dreimal mit Wasser. Nach Trocknung der vereinigten organischen Extrakte über Magnesiumsulfat und Chromatographie an Kieselgel mit Chloroform/Diethylether im Verhältnis 8/2 als Elutionsmittel isoliert man 72 mg (109 µmol, 52%) der Titelverbindung.
IR (Film): 3080, 3070, 3010, 3000, 2985, 1730, 1700, 1590, 1495, 1350, 1310, 1260, 1180, 1120, 1030, 920, 835 und 770 cm⁻¹.

### Beispiel 19:

### 5-[(3E/Z,1R,5R,6R,7R)-1-(4-Chlorphenylsulfonylaminomethyl)-6-[(1E,3S oder 3R)-3-hydroxy-3-cyclohexyl-prop-1-enyl]-7-hydroxy-bicyclo[3.3.0]oct-3-yliden]-pentansäure:

28,9 mg (50 µmol) der nach Beispiel 19a dargestellten Verbindung verseift man in Analogie zu Beispiel 1 und isoliert nach Aufarbeitung und Reinigung 27 mg (48 µmol, 96%) der Titelverbindung als farbloses Öl.
¹H-NMR (CDCl₃): δ= 0,80-1,05 (m,2H), 1,10-1,45 (m,6H), 1,60-1,80 (m,6H), 1,80-2,15 (m,6H), 2,20-2,40 (m,5H), 2,75 (d,2H), 3,75 (m,2H), 4,30-4,70 (breit,3H), 5,15 (s,1H), 5,40-5,50 (m, 2H), 6,05 (t,1H), 7,50 (d,H), 7,80 (d,2H).

### Beispiel 19a:

### 5-[(3E/Z,1R,5R,6R,7R)-1-(4-Chlorphenylsulfonylaminomethyl)-6-[(1E,3S oder 3R)-3-hydroxy-3-cyclohexyl-prop-1-enyl]-7-hydroxy-bicyclo[3.3.0]oct-3-yliden]-pentansäuremethylester:

42 mg (63 µmol) der nach 18b dargestellten Verbindung B setzt man in Analogie zu Beispiel 5a um und isoliert nach Aufarbeitung und Reinigung 28,9 mg (50 µmol, 79%) der Titelverbindung als farbloses Öl.
IR (Film): 3600-3100, 3090, 3010, 2990, 2940, 1725, 1610, 1585, 1450, 1335, 1250, 1160, 1070, 1020, 975, 830 und 750 cm⁻¹.

### Beispiel 20:

### 5-[(3E/Z,1R,5R,6R,7R)-1-(4-Chlorphenylsulfonylaminomethyl)-6-[(3R oder 3S)-3-hydroxy-3-cyclohexyl-prop-1-inyl]-7-hydroxy-bicyclo[3.3.0]oct-3-yliden]-pentansäure:

19,1 mg (33 µmol) der nach Beispiel 20a dargestellten Verbindung verseift man in Analogie zu Beispiel 1 und isoliert nach Aufarbeitung und Reinigung 16,8 mg (30 µmol, 91%) der Titelverbindung als farbloses Öl.
¹H-NMR (CDCl₃): δ= 0,98-1,30 (m,5H), 1,35-1,60 (m,2H), 1,60-1,90 (m,7H), 1,95-2,50 (m,11H), 2,80 (s,2H), 4,05 (q,1H), 4,15 (d,1H), 4,10-4,40 (breit,3H), 5,20 (s,1H), 5,90 (s,1H), 7,50 (d,2H), 7,80 (d,2H).

### Beispiel 20a:

### 5-[(3E/Z,1R,5R,6R,7R)-1-(4-Chlorphenylsulfonylaminomethyl)-6-[(3R oder 3S)-3-hydroxy-3-cyclo-hexyl-prop-1-inyl]-7-hydroxy-bicyclo[3.3.0]oct-3-yliden]-pentansäuremethylester:

21 mg (31 µmol) der nach 20b dargestellten Verbindung setzt man in Analogie zu Beispiel 5a um und isoliert nach Aufarbeitung und Reinigung 18,1 mg (30 µmol, 97%) der Titelverbindung als farbloses Öl.
IR (Film): 3600-3100, 3020, 3010, 3000, 2890, 1720, 1600, 1495, 1440, 1360, 1320, 1260, 1240, 1190, 1130, 1050, 870 und 735 cm⁻¹.

### Beispiel 20b:

### 5-[(3E/Z,1R,5R,6R,7R)-1-(4-Chlorphenylsulfonylaminomethyl)-6-[(1Z,3R oder 3S)-2-brom-3-hydroxy-3-cyclohexyl-prop-1-enyl]-7-hydroxy-bicyclo[3.3.0]oct-3-yliden]-pentansäuremethylester:

29 mg (39 µmol) der nach 20c dargestellten Verbindung A setzt man in Analogie zu Beispiel 1a um und isoliert nach Aufarbeitung und Reinigung 21,2 mg (32 µmol, 82%) der Titelverbindung als farbloses Öl.
IR (Film): 3600-3090, 3040, 3010, 3000, 2980, 1725, 1595, 1495, 1310, 1280, 1220, 1180, 1050, 1020, 870, 790 und 740 cm⁻¹

### Beispiel 20c:

### 5-[(3E/Z,1R,5R,6R,7R)-1-(4-Chlorphenylsulfonylaminomethyl)-6-[(1Z,3R oder 3S)-2-brom-3-hydroxy-3-cyclohexyl-prop-1-enyl]-7-(tetrahydropyran-2-yloxy)-bicyclo[3.3.0]oct-3-yliden]-pentansäuremethylester (A) und 5-[(3E/Z,1R,5R,6R,7R)-1-(4-Chlorphenylsulfonylaminomethyl)-6-[(1Z,3S oder 3R)-2-brom-3-hydroxy-3-cyclohexyl-prop-1-enyl]-7-(tetrahydropyran-2-yloxy)-bicyclo[3.3.0]oct-3-yliden]-pentansäuremethylester (B):

108 mg (145 µmol) der nach Beispiel 20d dargestellten Verbindung setzt man in Analogie zu Beispiel 1b um und isoliert nach Aufarbeitung und Reinigung 51 mg (69 µmol, 48%) einer unpolaren Komponente, der man die Struktur B zuordnet, sowie 29 mg (39 µmol, 27%) einer polaren Komponente, der man die Struktur A zuordnet, jeweils als farbloses Öl.
IR (Film) von A und B: 3600-3100, 3020, 3010, 3000, 2880, 1730, 1585, 1495, 1310, 1280, 1220, 1170, 1050, 1015, 870, 820, 790 und 735 cm⁻¹

### Beispiel 20d:

### 5-[(3E/Z,1R,5R,6R,7R)-1-(4-Chlorphenylsulfonylaminomethyl)-6[(1Z)-2-brom-3-oxo-3-cyclohexyl-prop-1-enyl]-7-(tetrahydropyran-2-yloxy)-bicyclo[3.3.0]oct-3-yliden]-pentansäuremethylester:

Zu der Aufschlämmung von 8,8 mg Natriumhydrid-Dispersion (80%) in 1,9 ml wasserfreiem Tetrahydrofuran tropft man bei Raumtemperatur unter einer Atmosphäre aus trockenem Argon die Lösung von 71,4 mg Dimethyl-(2-oxo-2-cyclohexylethyl)-phosphonat in 0,7 ml wasserfreiem Tetrahydrofuran und rührt 10 Minuten. Auschließend wird auf 0°C abgekühlt und mit 57,8 mg N-Bromsuccinimid versetzt. Man läßt eine Stunde reagieren und tropft dann 112,6 mg (203 µmol) des nach Beispiel 1d dargestellten Rohaldehyds gelöst in 0,7 ml Tetrahydrofuran hinzu. Man läßt auf 23°C erwärmen, rührt 5,5 Stunden, gießt dann auf 10%ige Ammoniumchloridlösung und extrahiert mehrfach mit Diethylether. Die vereinigten organischen Phasen wäscht man mit Wasser, trocknet über Magnesiumsulfat und reinigt den nach Lösungsmittelabzug erhaltenen Rückstand durch präparative Plattenchromatographie mit n-Hexan/Essigester im Verhältnis 7/3 als Elutionsmittel. Isoliert werden 108 mg (146 µmol, 72%) der Titelverbindung als farbloses Öl.
IR (Film): 3290, 3010, 2950, 1725, 1685, 1610, 1595, 1465, 1325, 1250, 1160, 1010, 975, 840, 785 und 735 cm⁻¹.

### Beispiel 21:

### 5-[(3E/Z,1R,5R,6R,7R)-1-(4-Chlorphenylsulfonylaminomethyl)-6-[(3S oder 3R)-3-hydroxy-3-cyclohexyl-prop-1-inyl]-7-hydroxy-bicyclo[3.3.0]oct-3-yliden]-pentansäure:

36,2 mg (63 µmol) der nach Beispiel 21a dargestellten Verbindung verseift man in Analogie zu Beispiel 1 und isoliert nach Aufarbeitung und Reinigung 32,7 mg (58 µmol, 92%) der Titelverbindung als farbloses Öl.
¹H-NMR (CDCl₃): δ= 0,95-1,30 (m,5H), 1,35-1,55 (m,1H), 1,60-1,90 (m,7H), 1,95-2,50 (m,12H), 2,80 (d,2H), 4,05 (q,1H), 4,15 (d,1H), 4,40-4,90 (breit 3H), 5,20 (1H), 5,95 (s,1H), 7,50 (d,2H), 7,80 (d,2H).

### Beispiel 21a:

### 5-[(3E/Z,1R,5R,6R,7R)-1-(4-Chlorphenylsulfonylaminomethyl)-6-[(3S oder 3R)-3-hydroxy-3-cyclohexyl-prop-1-inyl]-7-hydroxy-bicyclo[3.3.0]oct-3-yliden]-pentansäuremethylester:

45,5 mg (69 µmol) der nach 21b dargestellten Verbindung setzt man in Analogie zu Beispiel 5a um und isoliert nach Aufarbeitung und Reinigung 36,2 mg (63 µmol, 91%) der Titelverbindung als farbloses Öl.
IR (Film): 3600-3100, 3020, 3010, 3000, 2890, 1720, 1600, 1495, 1440, 1360, 1320, 1260, 1240, 1190, 1130, 1050, 870 und 735 cm⁻¹.

### Beispiel 21b:

### 5-[(3E/Z,1R,5R,6R,7R)-1-(4-Chlorphenylsulfonylaminomethyl)-6-[(1Z,3S oder 3R)-2-brom-3-hydroxy-3-cyclohexyl-prop-1-enyl]-7-hydroxy-bicyclo[3.3.0]oct-3-yliden]-pentansäuremethylester:

51 mg (69 µmol) der nach 20c dargestellten Verbindung B setzt man in Analogie zu Beispiel 1a um und isoliert nach Aufarbeitung und Reinigung 45,5 mg (69 µmol, 100%) der Titelverbindung als farbloses Öl.
IR (Film): 3600-3090, 3040, 3010, 3000, 2980, 1725, 1595, 1495, 1310, 1280, 1220, 1180, 1050, 1020, 870, 790 und 740 cm⁻¹

### Beispiel 22:

### 5-[(3E/Z,1R,5R,6R,7R)-1-(4-Chlorphenylsulfonylaminomethyl)-6-[(1E,3R oder 3S,4RS)-3-hydroxy-4-pheny-but-1-enyl]-7-hydroxy-bicyclo[3.3.0]oct-3-yliden]-pentansäure:

14,7 mg (24 µmol) der nach Beispiel 22a dargestellten Verbindung verseift man in Analogie zu Beispiel 1 und isoliert nach Aufarbeitung und Reinigung 10,2 mg (17 µmol, 70%) der Titelverbindung als farbloses Öl.
¹H-NMR (CDCl₃): δ= 1,20 (d,3H), 1,35-1,45 (m,1H), 1,60-1,75 (m,3H), 1,90-2,15 (m,5H), 2,15-2,40 (m,5H), 2,70-2,85 (m,3H), 3,40-3,90 (breit,3H), 3,75 (q,1H), 4,15 (m,1H), 5,20 (s,1H), 5,55 (m,2H), 5,80 (s,1H), 7,15-7,35 (m,5H), 7,50 (d,2H), 7,75 (d,2H).

### Beispiel 22a:

### 5-[(3E/Z,1R,5R,6R,7R)-1-(4-Chlorphenylsulfonylaminomethyl)-6-[(1E,3R oder 3S,4RS)-3-hydroxy-4-phenyl-but-1-enyl]-7-hydroxy-bicyclo[3.3.0]oct-3-yliden]-pentansäuremethylester:

22,4mg (32 µmol) der nach 22b dargestellten Verbindung A setzt man in Analogie zu Beispiel 5a um und isoliert nach Aufarbeitung und Reinigung 14,7 mg (24 µmol, 75%) der Titelverbindung als farbloses Öl.
IR (Film): 3600-3100, 3090, 3010, 3000, 2990, 2980, 2940, 1725, 1610, 1585, 1450, 1335, 1250, 1150, 1110, 1070, 1030, 970, 830 und 735 cm⁻¹.

### Beispiel 22b:

### 5-[(3E/Z,1R,5R,6R,7R)-1-(4-Chlorphenylsulfonylaminomethyl)-6-[(1E,3R oder 3S,4RS)-3-hydroxy-4-phenyl-but-1-enyl]-7-(tetrahydropyran-2-yloxy)-bicyclo[3.3.0]oct-3-yliden]-pentansäuremethylester (A) und 5-[(3E/Z,1R,5R,6R,7R)-1-(4-Chlorphenylsulfonylaminomethyl)-6-[(1E,3S oder 3R,4RS)-3-hydroxy-4-phenyl-but-1-enyl]-7-(tetrahydropyran-2-yloxy)-bicyclo[3.3.0]oct-3-yliden]-pentansäuremethylester (B):

83 mg (121 µmol) der nach Beispiel 22c dargestellten Verbindung setzt man in Analogie zu Beispiel 1b um und isoliert nach Aufarbeitung und Reinigung 51,4 mg (75 µmol, 62%) einer unpolaren Komponente, der man die Struktur B zuordnet, sowie 22,4 mg (33 µmol, 27%) einer polaren Komponente, der man die Stur A zuordnet, jeweils als farbloses Öl.
IR (Film) von A und B: 3600-3100, 3060, 3000, 2990, 1725, 1595, 1450, 1330, 1250, 1165, 1080, 830, 740 und 700 cm⁻¹

### Beispiel 22c:

### 5-[(3E/Z,1R,5R,6R,7R)-1-(4-Chlorphenylsulfonylaminomethyl)-6-[(1E,4RS)-3-oxo-4-phenyl-but-1-enyl]-7-(tetrahydropyran-2-yloxy)-bicyclo[3.3.0]oct-3-yliden]-pentansäuremethylester:

Zu der Aufschlämmung von 7,2 mg Natriumhydrid-Dispersion (80%ig) in 1,8 ml wasserfreiem Tetrahydrofuran gibt man unter Argon bei Raumtemperatur 63,6 mg Dimethyl-(2-oxo-3RS-phenyl-propyl)-phosphonat. Nach Beendigung der Gasentwicklung kühlt man auf -30°C und versetzt mit 110,9 mg des nach Beispiel 1d dargestellten Rohaldehyds gelöst in 0,7 ml Tetrahydrofuran. Anschließend läßt man bei einer Temperatur zwischen -25°C und 2°C 3,5 Stunden reagieren. Zur Aufarbeitung gibt man 0,11 ml Essigsäure zu, läßt auf Raumtemperatur kommen, verdünnt mit Diethylether, wäscht einmal mit 10%iger Ammoniumchloridlösung und dreimal mit Wasser. Nach Trocknung der vereinigten organischen Phasen über Magnesiumsulfat und Chromatographie an Kieselgel mit Chloroform/Diethylether im Verhältnis 8/2 isoliert man 83 mg (121 µmol, 61%) der Titelverbindung.
IR (Film): 3070, 3065, 3010, 3000, 2985, 1725, 1700, 1590, 1510, 1360, 1330, 1250, 1165, 1010, 835, 780, 735 und 700 cm⁻¹.

### Beispiel 23:

### 5-[(3E/Z,1R,5R,6R,7R)-1-(4-Chlorphenylsulfonylaminomethyl)-6-[(1E,3S oder 3R,4RS)-3-hydroxy-4-phenyl-but-1-enyl]-7-hydroxy-bicyclo[3.3.0]oct-3-yliden]-pentansäure:

40,2 mg (67 µmol) der nach Beispiel 23a dargestellten Verbindung verseift man in Analogie zu Beispiel 1 und isoliert nach Aufarbeitung und Reinigung 32,2 mg (55 µmol, 82%) der Titelverbindung als farbloses Öl.
¹H-NMR (CDCl₃): δ= 1,15 (d,3H), 1,25-1,40 (m,1H), 1,60-1,80 (m,3H), 1,80-2,10 (m,5H), 2,10-2,35 (m,5H), 2,60-2,80 (m,3H), 3,70 (q,1H), 3,95 (m,1H), 4,20-4,60 (breit,3H), 5,20 (s,1H), 5,45 (m,2H), 6,00 (s,1H), 7,1 (t,1H), 7,15 (d,2H), 7,25 (m,2H), 7,50 (d,2H), 7,70 (d,2H).

### Beispiel 23a:

### 5-[(3E/Z,1R,5R,6R,7R)-1-(4-Chlorphenylsulfonylaminomethyl)-6-[(1E,3S oder 3R,4RS)-3-hydroxy-4-phenyl-but-1-enyl]-7-hydroxy-bicyclo[3.3.0]oct-3-yliden]-pentansäuremethylester:

51,4mg (75 µmol) der nach 22b dargestellten Verbindung B setzt man in Analogie zu Beispiel 5a um und isoliert nach Aufarbeitung und Reinigung 40,2 mg (66 µmol, 88%) der Titelverbindung als farbloses Öl.
IR (Film): 3600-3100, 3090, 3010, 3000, 2990, 2980, 2940, 1725, 1610, 1585, 1450, 1335, 1250, 1150, 1110, 1070, 1030, 970, 830 und 735 cm⁻¹.

### Beispiel 24:

### 5-[(3E/Z,1R,5R,6R,7R)-1-(4-Chlorphenylsulfonylaminomethyl)-6-[(1E,6E,3R oder 3S,-4RS)-3-hydroxy-4-methyl-7-chloro-oct-1,6-dien-1-yl]-7-hydroxy-bicyclo[3.3.0]oct-3-yliden]-pentansäure:

15,4 mg (30 µmol) der nach Beispiel 24a dargestellten Verbindung verseift man in Analogie zu Beispiel 1 und isoliert nach Aufarbeitung und Reinigung 14,1 mg (23 µmol, 77%) der Titelverbindung als farbloses Öl.
¹H-NMR (CDCl₃): δ= 0,90 (t,3H), 1,45 (t,1H), 1,60-1,80 (m,4H), 1,90-2,10 (m,6H), 2,10 (s,3H), 2,20-2,40 (m,6H), 2,75 (d,2H), 3,50-4,00 (breit,3H), 3,75 (q,1H), 4,00 (m, 1H), 5,20 (s,1H), 5,45 (t,1H), 5,60 (s,2H), 5,90 (s,1H), 7,50 (d,2H), 7,80 (d,2H).

### Beispiel 24a:

### 5-[(3E/Z,1R,5R,6R,7R)-1-(4-Chlorphenylsulfonylaminomethyl)-6-[(1E,6E,3R oder 3S,-4RS)-3-hydroxy-4-methyl-7-chloro-oct-1,6-dien-1-yl]-7-hydroxy-bicyclo[3.3.0]oct-3-yliden]-pentansäuremethylester:

21,6mg (31 µmol) der nach 24b dargestellten Verbindung A setzt man in Analogie zu Beispiel 5a um und isoliert nach Aufarbeitung und Reinigung 15,4 mg (30 µmol, 97%) der Titelverbindung als farbloses Öl.
IR (Film): 3600-3100, 3010, 3000, 2990, 2980, 2940, 1725, 1585, 1450, 1340, 1260, 1210, 1150, 1110, 1070, 1030, 970, 940, 830 und 750 cm⁻¹.

### Beispiel 24b:

### 5-[(3E/Z,1R,5R,6R,7R)-1-(4-Chlorphenylsulfonylaminomethyl)-6-[(1E,6E,3R oder 3S,-4RS)-3-hydroxy-4-methyl-7-chloro-oct-1,6-dien-1-yl]-7-(tetrahydropyran-2-yloxy)-bicyclo[3.3.0]oct-3-yliden]-pentansäuremethylester (A) und 5-[(3E/Z,1R,5R,6R,7R)-1-(4 Chlorphenylsulfonylaminomethyl)-6-[(1E,6E,3S oder 3R,4RS)-3-hydroxy-4-methyl-7-chloro-oct-1,6-dien-1-yl]-7-(tetrahydropyran-2-yloxy)-bicyclo[3.3.0]oct-3-yliden]-pentansäuremethylester (B):

71 mg (102 µmol) der nach Beispiel 24c dargestellten Verbindung setzt man in Analogie zu Beispiel 1b um und isoliert nach Aufarbeitung und Reinigung 45 mg (65 µmol, 64%) einer unpolaren Komponente, der man die Struktur B zuordnet, sowie 21,6 mg (31 µmol, 30%) einer polaren Komponente, der man die Struktur A zuordnet, jeweils als farbloses Öl.
IR (Film) von A und B: 3600-3100, 3020, 3010, 3000, 2990, 1725, 1590, 1450, 1340, 1300, 1290, 1250, 1160, 1080, 830, 740 und 700 cm⁻¹

### Beispiel 24c:

### 5-[(3E/Z,1R,5R,6R,7R)-1-(4-Chlorphenylsulfonylaminomethyl)-6-[(1E,6E,4RS)-3-oxo-4-methyl-7-chloro-oct-1,6-dien-1-yl]-7-(tetrahydropyran-2-yloxy)-bicyclo[3.3.0]oct-3-yliden] -pentansäuremethylester:

Zu der Aufschlämmung von 7,1 mg Natriumhydrid-Dispersion (80%ig) in 1,8 ml wasserfreiem Tetrahydrofuran gibt man unter Argon bei Raumtemperatur 66,1 mg Dimethyl[(5E,3RS)-2-oxo-3-methyl-6-chloro-hept-5-en-1-yl]-phosphonat. Nach Beendigung der Gasentwicklung kühlt man auf -30°C und versetzt mit 110 mg des nach Beispiel 1d dargestellten Rohaldehyds gelöst in 0,7 ml Tetrahydrofuran. Anschließend läßt man bei einer Temperatur zwischen -20°C und 7°C 3,5 Stunden reagieren. Zur Aufarbeitung gibt man 0,11 ml Essigsäure zu, läßt auf Raumtemperatur kommen, verdünnt mit Diethylether, wäscht einmal mit 10%iger Ammoniumchloridlösung und dreimal mit Wasser. Nach Trocknung der vereinigten organischen Phasen über Magnesiumsulfat und Chromatographie an Kieselgel mit Chloroform/Diethylether im Verhältnis 8/2 isoliert man 71 mg (102 µmol, 51%) der Titelverbindung.
IR (Film): 3060, 3010, 3000, 2995, 1725, 1700, 1580, 1510, 1360, 1330, 1300, 1250, 1160, 1010, 835, 780, 740 und 700 cm⁻¹.

### Beispiel 25:

### 5-[(3E/Z,1R,5R,6R,7R)-1-(4-Chlorphenylsulfonylaminomethyl)-6-[(1E,6E,3S oder 3R,-4RS)-3-hydroxy-4-methyl-7-chloro-oct-1,6-dien-1-yl]-7-hydroxy-bicyclo[3.3.0]oct-3-yliden]-pentansäure:

33,7 mg (44 µmol) der nach Beispiel 25a dargestellten Verbindung verseift man in Analogie zu Beispiel 1 und isoliert nach Aufarbeitung und Reinigung 26,7 mg (44 µmol, 68%) der Titelverbindung als farbloses Öl.
¹H-NMR (CDCl₃): δ= 0,90 (dd,3H), 1,40 (t,1H), 1,60-1,80 (m,4H), 1,85-2,10 (m,6H), 2,10 (s,3H), 2,20-2,35 (m,3H), 2,75 (d,2H), 3,80 (m,2H), 4,10-4,50 (breit,3H), 5,20 (s,1H), 5,50 (m,3H), 6,10 (s,1H), 7,50 (d,2H), 7,80 (d,2H).

### Beispiel 25a:

### 5-[(3E/Z,1R,5R,6R,7R)-1-(4-Chlorphenylsulfonylaminomethyl)-6-[(1E,6E,3S oder 3R,-4RS)-3-hydroxy-4-methyl-7-chloro-oct-1,6-dien-1-yl]-7-hydroxy-bicyclo[3.3.0]oct-3-yliden]-pentansäuremethylester:

45 mg (65 µmol) der nach 24b dargestellten Verbindung B setzt man in Analogie zu Beispiel 5a um und isoliert nach Aufarbeitung und Reinigung 33,7 mg (65 µmol, 100%) der Titelverbindung als farbloses Öl.
IR (Film): 3600-3100, 3010, 3000, 2990, 2980, 2940, 1725, 1585, 1450, 1340, 1260, 1210, 1150, 1110, 1070, 1030, 970, 940, 830 und 750 cm⁻¹.

### Beispiel 26:

### 5-[(3E/Z,1R,5R,6R,7R)-1-(4-Chlorphenylsulfonylaminomethyl)-6-[(1E,3R oder 3S)-3-hydroxy-5-phenyl-pent-1-enyl]-7-hydroxy-bicyclo[3.3.0]oct-3-yliden]-pentansäure:

17 mg (28 µmol) der nach Beispiel 26a dargestellten Verbindung verseift man in Analogie zu Beispiel 1 und isoliert nach Aufarbeitung und Reinigung 15 mg (25 µmol, 89%) der Titelverbindung als farbloses Öl.
¹H-NMR (CDCl₃): δ= 1.40 (t,1H), 1,60-2,10 (m,10H), 2,20-2,35 (m,5H), 2,65 (m,2H), 2,75 (d,2H), 3,70 (q,1H), 9,90-4,20 (breit,3H), 4,15 (m,1H), 5,20 (s,1H), 5,60 (m,2H), 6,00 (s,1H), 7,15 (d,3H), 7,25 (m,2H), 7,45 (d,2H), 7,80 (d,2H).

### Beispiel 26a:

### 5-[(3E/Z,1R,5R,6R,7R)-1-(4-Chlorphenylsulfonylaminomethyl)-6[(1E,3R oder 3S)-3-hydroxy-5-phenyl-pent-1-enyl]-7-hydroxy-bicyclo[3.3.0]oct-3-yliden]-pentansäuremethylester:

20,5 mg (30 µmol) der nach 26b dargestellten Verbindung A setzt man in Analogie zu Beispiel 5a um und isoliert nach Aufarbeitung und Reinigung 17 mg (28 µmol, 93%) der Titelverbindung als farbloses Öl.
IR (Film): 3600-3100, 3010, 3000, 2990, 2970, 2940, 1720, 1585, 1440, 1340, 1240, 1210, 1180, 1150, 1110, 1060, 950, 940, 810 und 735 cm⁻¹.

### Beispiel 26b:

### 5-[(3E/Z,1R,5R,6R,7R)-1-(4-Chlorphenylsulfonylaminomethyl)-6-[(1E,3R oder 3S)-3-hydroxy-5-phenyl-pent-1-enyl]-7-(tetrahydopyran-2-yloxy)-bicyclo[3.3.0]oct-3-yliden]-pentansäuremethylester (A) und 5-[(3E/Z,1R,5R,6R,7R)-1-(4-Chlorphenylsulfonylaminomethyl)-6[(1E,3S oder 3R)-3-hydroxy-5-phenyl-pent-1-enyl]-7-(tetrahydropyran-2-yloxy)-bicyclo[3.3.0]oct-3-yliden]-pentansäuremethylester (B):

71 mg (104 µmol) der nach Beispiel 26c dargestellten Verbindung setzt man in Analogie zu Beispiel 1b um und isoliert nach Aufarbeitung und Reinigung 42 mg (61 µmol, 59%) einer unpolaren Komponente, der man die Struktur B zuordnet, sowie 20,5 mg (30 µmol, 29%) einer polaren Komponente, der man die Struktur A zuordnet, jeweils als farbloses Öl.
IR (Film) von A und B: 3600-3100, 3020, 3010, 3000, 2990, 1725, 1580, 1480, 1340, 1310, 1290, 1230, 1160, 1080, 1050, 820, 740 und 730 cm⁻¹

### Beispiel 26c:

### 5-[(3E/Z,1R,5R,6R,7R)-1-(4-Chlorphenylsulfonylaminomethyl)-6-[(1E)-3-oxo-5-phenylpent-1-enyl]-7-(tetrahydopyran-2-yloxy)-bicyclo[3.3.0]oct-3-yliden]-pentansäuremethylester:

Zu der Aufschlämmung von 7,4 mg Natriumhydrid-Dispersion (80%ig) in 1,9 ml wasserfreiem Tetrahydrofuran gibt man unter Argon bei Raumtemperatur 65,4 mg Dimethyl-(2-oxo-4-phenyl-butyl)-phosphonat. Nach Beendigung der Gasentwicklung kühlt man auf -30°C und versetzt mit 114,1 mg des nach Beispiel 1d dargestellten Rohaldehyds gelöst in 0,7 ml Tetrahydrofuran. Anschließend läßt man bei einer Temperatur zwischen -20°C und 7°C 3,5 Stunden reagieren. Zur Aufarbeitung gibt man 0,11 ml Essigsäure zu, läßt auf Raumtemperatur kommen, verdünnt mit Diethylether, wäscht einmal mit 10%iger Ammoniumchloridlösung und dreimal mit Wasser. Nach Trocknung der vereinigten organischen Phasen über Magnesiumsulfat und Chromatographie an Kieselgel mit Chloroform/Diethylether im Verhältnis 8/2 isoliert man 71,3 mg (104 µmol, 50%) der Titelverbindung.
IR (Film): 3050, 3020, 3010, 3000, 2990, 1725, 1700, 1590, 1490, 1330, 1310, 1300, 1250, 1180, 1160, 1000, 840, 760, 740 und 700 cm⁻¹.

### Beispiel 27:

### 5-[(3E/Z,1R,5R,6R,7R)-1-(4-Chlorphenylsulfonylaminomethyl)-6-[(1E,3S oder 3R)-3-hydroxy-5-phenyl-pent-1-enyl]-7-hydroxy-bicyclo[3.3.0]oct-3-yliden]-pentansäure:

27,8 mg (46 µmol) der nach Beispiel 27a dargestellten Verbindung verseift man in Analogie zu Beispiel 1 und isoliert nach Aufarbeitung und Reinigung 23,6 mg (40 µmol, 87%) der Titelverbindung als farbloses Öl.
¹H-NMR (CDCl₃): δ= 1,40 (m,1H), 1,60-2,15 (m,10H), 2,20-2,35 (m,5H), 2,65 (m,2H), 2,75 (d,2H), 3,80 (q,1H), 4,10 (q,1H), 4,15-4,50 (breit,3H), 5,20 (s,1H), 5,50 (m,2H),6,10 (t,1H), 7,20 (d,3H), 7,25 (m,2H), 7,45 (d,2H), 7,80 (d,2H).

### Beispiel 27a:

### 5-[(3E/Z,1R,5R,6R,7R)-1-(4-Chlorphenylsulfonylaminomethyl)-6-[(1E,3S oder 3R)-3-hydroxy-5-phenyl-pent-1-enyl]-7-hydroxy-bicyclo[3.3.0]oct-3-yliden]-pentansäuremethylester:

42 mg (61 µmol) der nach 26b dargestellten Verbindung B setzt man in Analogie zu Beispiel 5a um und isoliert nach Aufarbeitung und Reinigung 27,8 mg (46 µmol, 75%) der Titelverbindung als farbloses Öl.
IR (Film): 3600-3100, 3010, 3000, 2990, 2970, 2940, 1720, 1585, 1440, 1340, 1240, 1210, 1180, 1150, 1110, 1060, 950, 940, 810 und 735 cm⁻¹.

## Patentansprüche

1. 9-Substituierte Bicyclo[3.3.0]octan-Derivate der Formel I, 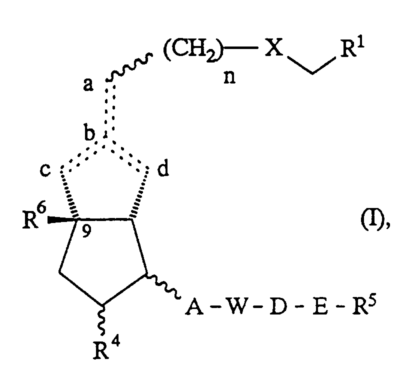 sowie deren Enantiomere,
worin
zwischen den Kohlenstoffatomen der Zentren a-b oder b-c oder b-d maximal eine Doppelbindung liegt,
R¹ 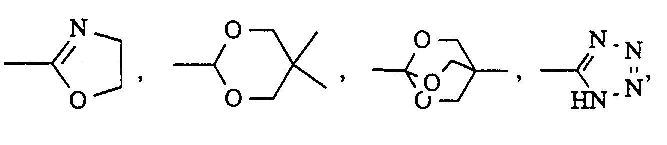 COOR², wobei R² Wasserstoff oder gegebenenfalls durch Halogen, Phenyl, C₁-C₄-Alkoxy oder Di-(C₁-C₄)-alkylamino substituiertes C₁-C₁₀-Alkyl, C₃-C₁₀-Cycloalkyl, C₇-C₁₆-Aralkyl, durch Y substituiertes Phenacyl oder C₆-C₁₂-Aryl oder einen 5- oder 6- gliedrigen heterocyclischen Rest mit wenigstens einem N-, O- oder S-Atom bedeuten kann, oder -CONHR³ mit R³ in der Bedeutung Wasserstoff, C₁-C₁₀-Alkanoyl oder C₁-C₁₀-Alkansulfonyl sein kann,
X eine CH₂-Gruppe, ein Sauerstoffatom oder eine O-CH₂-CH₂-Gruppe,
n 0 bis 3,
R⁴ ein Wasserstoffatom, Halogen, eine freie oder funktionell abgewandelte Hydroxygruppe, wobei die Hydroxygruppe α- oder β-ständig sein kann,
R⁶-(CH₂)_{q}-R⁷ oder ―C≡C―(CH₂)_{q}―R⁷,
q 1 bis 5,
R⁷ 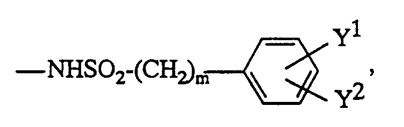 m 0 bis 2
A eine cis- oder trans CH=CH- oder eine ―C≡C― Gruppe,
W eine 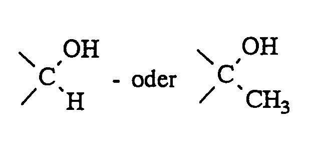 -Gruppe, wobei die OH-Gruppe α- oder β-ständig sein kann,
D und E zusammen eine Bindung oder
D eine Bindung, oder C₁-C₁₀-Alkylen,
E eine Bindung, eine ―C≡C― Gruppe, eine -CR⁹=CR¹⁰-Gruppe, wobei R⁹ und R¹⁰ gleich oder verschieden sind und Wasserstoff, Chlor oder Brom, oder eine C₁-C₅-Alkylgruppe bedeuten, oder 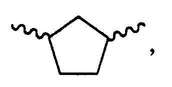 R⁵ ein Wasserstoffatom, durch Y substituiertes C₁-C₁₀-Alkyl, C₃-C₁₀-Cycloalkyl oder C₆-C₁₂-Aryl,
Y¹ und Y² gleich oder verschieden sind und Y bedeuten,
Y Wasserstoff, Halogen, N₃, NH₂, CN, CF₃, OR⁸, NO₂, COOR⁸ oder C₁-C₁₀-Alkyl,
R⁸ Wasserstoff, C₁-C₁₀-Alkyl, gegebeneufalls durch Halogen substituiertes C₆-C₁₂-Aryl oder C₇-C₁₆-Aralkyl sein kann und, falls R² Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

2. Arzneimittel bestehend aus einer oder mehreren Verbindungen des Anspruchs 1 und üblichen Hilfs-, Träger- und Zusatzstoffen.

3. Verfahren zur Herstellung von Bicyclo[3.30]octan-Derivaten der Formel I, dadurch gekennzeichnet, daß man die Hydroxy-Verbindung der Formel II 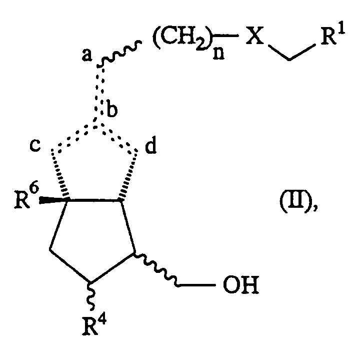 worin a-b, b-c, b-d, R⁶, n und X die oben angegebenen Bedeutungen haben und R¹ eine -COOR²-Estergruppe mit R² in der mit Ausnahme von Wasserstoff oben angegebenen Bedeutung darstellt, zu einem Aldehyd der Formel III 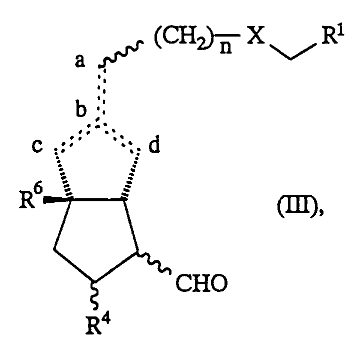 oxidiert und mit einer Verbindung der Formel IV 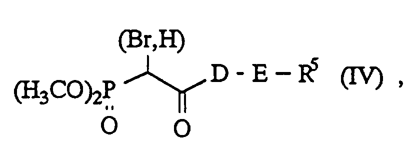 worin D, E und R⁵ die oben genannten Bedeutungen haben, in eine Verbindung der Formel V, worin a-b, b-c, b-d, R⁶, R⁴, D, E, R⁵, n, und X die oben angegebenen Bedeutungen haben und R¹ eine -COOR²-Estergruppe mit R² in der mit Ausnahme von Wasserstoff oben angegebenen Bedeutung darstellt, überführt, das gebildete Keton reduziert und gegebenenfalls Bromwasserstoff eliminiert, die erhaltenen Ester verseift, mit physiologisch 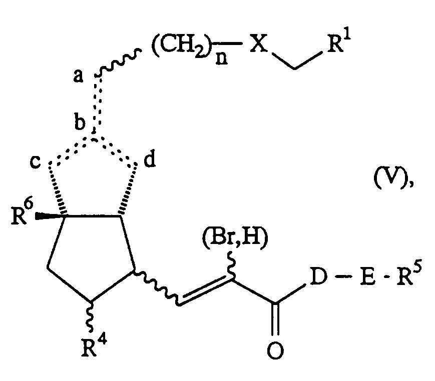 verträglichen Basen in deren Salze überführt, mit α-, β- oder γ- Cyclodextrin zu einem Clathrat umwandelt oder mit Liposomen verkapselt.

## Claims

1. 9-substituted bicyclo[3.3.0]octane derivatives of formula I 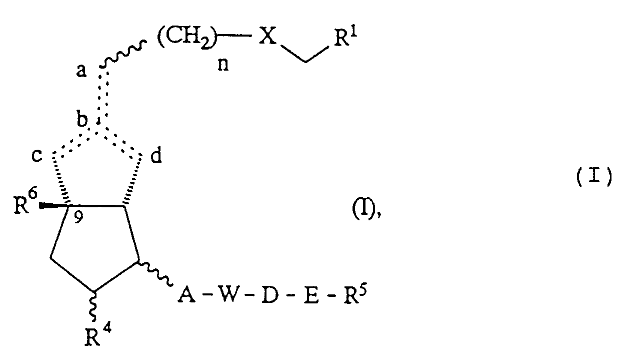 and their enantiomers,
wherein
there is a maximum of one double bond between the carbon atoms of the centres a-b and b-c and b-d,
R¹ may be 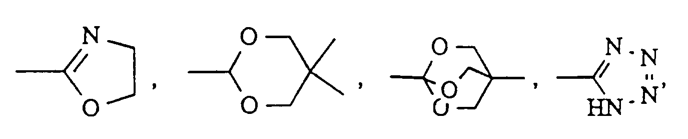 COOR², wherein R² may be hydrogen or optionally halo-, phenyl-, C₁-C₄alkoxy- or di(C₁-C₄)alkylamino-substituted C₁-C₁₀alkyl, C₃-C₁₀cycloalkyl or C₇-C₁₆aralkyl, Y-substituted phenacyl or C₆-C₁₂aryl or a 5- or 6-membered heterocyclic radical having at least one N, O or S atom, or R¹ may be -CONHR³ wherein R³ represents hydrogen, C₁-C₁₀alkanoyl or C₁-C₁₀alkanesulphonyl,
X is a CH₂ group, an oxygen atom or an O-CH₂-CH₂ group,
n is from 0 to 3,
R⁴ is a hydrogen atom, halogen, or a free or functionally modified hydroxy group, it being possible for the hydroxy group to be in the α- or β-configuration,
R⁶ is -(CH₂)_{q}-R⁷ or ―C≡C―(CH₂)_{q}―R⁷,
g is from 1 to 5,
R⁷ is 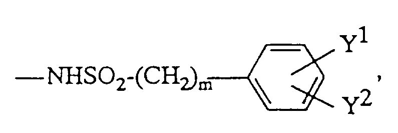 m is from 0 to 2,
A is a cis- or trans-CH=CH- or a ―C≡C― group,
W is a 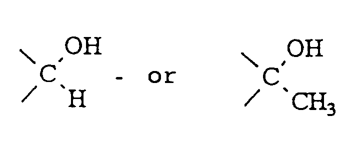 group, it being possible for the OH group to be in the α- or β-configuration,
D and E together represent a bond, or
D is a bond, or C₁-C₁₀alkylene,
E is a bond, a ―C≡C― group, a -CR⁹=CR¹⁰ group, wherein R⁹ and R¹⁰ are the same or different and represent hydrogen, chlorine or bromine, or a C₁-C₅alkyl group, or E is 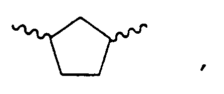 R⁵ is a hydrogen atom, Y-substituted C₁-C₁₀alkyl, C₃-C₁₀cycloalkyl or C₆-C₁₂aryl,
Y¹ and Y² are the same or different and represent Y,
Y is hydrogen, halogen, N₃, NH₂, CN, CF₃, OR⁸, NO₂, COOR⁸ or C₁-C₁₀alkyl,
R⁸ may be hydrogen, C₁-C₁₀alkyl, optionally halo-substituted C₆-C₁₂aryl or C₇-C₁₆aralkyl, and when R² represents hydrogen, salts thereof with physiologically tolerable bases, and the α-, β- or γ-cyclodextrin clathrates, and compounds of formula I encapsulated with liposomes.

2. Medicaments comprising one or more compounds of claim 1 and conventional excipients, carriers and additives.

3. Process for the preparation of bicyclo[3.3.0]octane derivatives of formula I, characterised in that the hydroxy compound of formula II 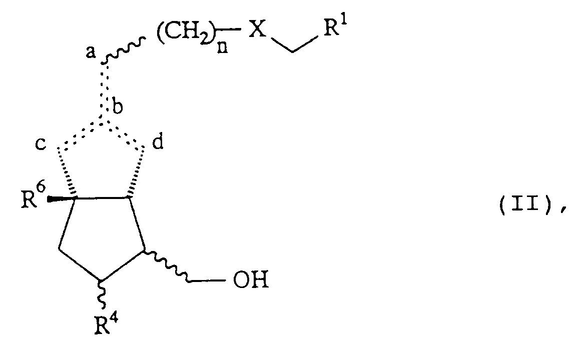 wherein a-b, b-c, b-d, R⁶, n and X are as defined above and R¹ is a -COOR² ester group wherein R² has the meanings given above with the exception of hydrogen, is oxidised to form an aldehyde of formula III 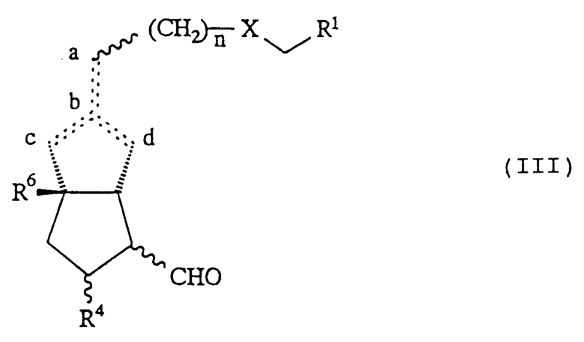 and is converted by means of a compound of formula IV 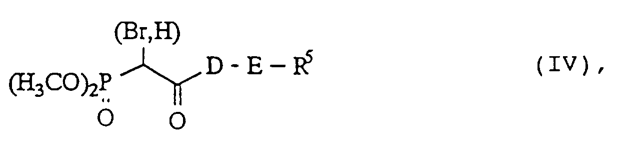 wherein D, E and R⁵ are as defined above, into a compound of formula V 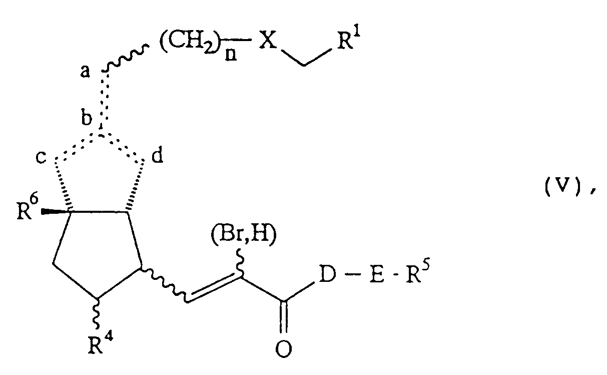 wherein a-b, b-c, b-d, R⁶, R⁴, D, E, R⁵ n and X are as defined above and R¹ is a -COOR² ester group wherein R² has the meanings given above with the exception of hydrogen, the resulting ketone is reduced and any hydrogen bromide present is removed and the resulting esters are hydrolysed, are converted into their salts with physiologically tolerable bases, are converted into a clathrate with α-, β- or γ-cyclodextrin or are encapsulated with liposomes.

## Revendications

1. Dérivés de bicyclo [3.3.0]octane substitués en position 9 de formule I : 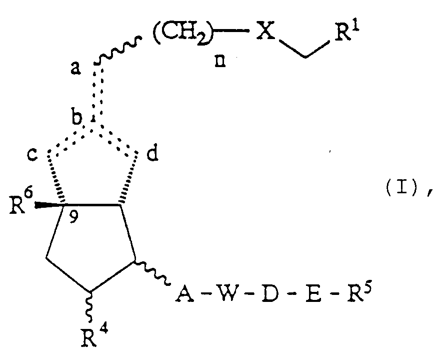 ainsi que leurs énantiomères,
dans laquelle
entre les atomes de carbone des centres a-b ou b-c ou b-d se trouve une double liaison au maximum, R¹ 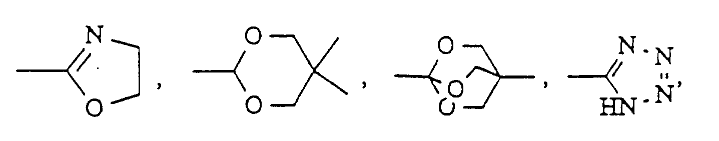 COOR²
R² pouvant représenter l'hydrogène ou aralkyle en C₇-C₁₆, cycloalkyle en C₃-C₁₀, alkyle en C₁-C₁₀ éventuellement substitué par halogène, par phényle, par alcoxy en C₁-C₄ ou par di(alkyl en C₁-C₄)-amino, aryle en C₆-C₁₂ ou phénacyle substitué par Y, ou un radical hétérocyclique à 5 ou 6 chaînons avec au moins un atome de N, O ou S, ou -CONHR³ avec R³ pouvant représenter l'hydrogène, alcanoyle en C₁-C₁₀ ou (alcane en C₁-C₁₀)-sulfonyle,
X peut représenter un groupe CH₂, un atome d'oxygène ou un groupe O-CH₂-CH₂,
n va de 0 à 3,
R⁴ peut représenter un atome d'hydrogène, halogène, un groupe hydroxy libre ou fonctionnellement modifié, le groupe OH pouvant être en position α ou β,
R⁶ représente -(CH₂)_{q}-R⁷ ou ―C≡C―(CH₂)_{q} R⁷,
q va de 1 à 5,
R⁷ 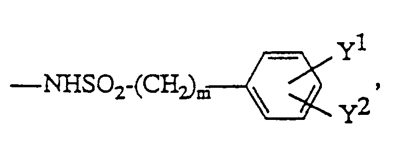 m va de 0 à 2
A représente un groupe cis ou trans CH=CH- ou un groupe ―C≡C―,
W représente un groupe 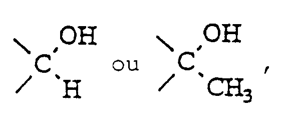 le groupe OH pouvant être en position α ou β,
D et E ensemble représentent une liaison ou
D représente une liaison ou alkylène en C₁-C₁₀,
E représente une liaison, un groupe ―C≡C― ou un groupe -CR⁹=CR¹⁰-, R⁹ et R¹⁰ pouvant être identiques ou différents et représenter l'hydrogène, le chlore ou le brome ou un groupe alkyle en C₁-C₅ ou 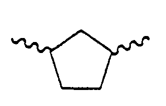 R⁵ représente un atome d'hydrogène, un aryle en C₆-C₁₂, un cycloalkyle en C₃-C₁₀ ou un alkyle en C₁-C₁₀ substitué par Y,
Y¹ et Y² sont identiques ou différents et représentent Y,
Y représente l'hydrogène, un halogène, N₃, NH₂, CN, CF₃, OR⁸, NO₂, COOR⁸ ou alkyle en C₁-C₁₀,
R⁸ peut être l'hydrogène, alkyle en C₁-C₁₀, ainsi qu'aralkyle en C₇-C₁₆ ou aryle en C₆-C₁₂ éventuellement substitués par halogène, et dans le cas où R² représente l'hydrogène, ses sels avec des bases physiologiquement compatibles, ainsi que des clathrates de cyclodextrines α, β ou γ, ainsi que des composés de formule I encapsulés dans des liposomes.

2. Médicaments composés d'un ou plusieurs composés de la revendication 1 et des adjuvants, véhicules et additifs usuels.

3. Procédé pour la préparation de dérivés de bicyclo[3.3.0]octane de formule I, caractérisé en ce qu'on oxyde le composé hydroxy de formule II : 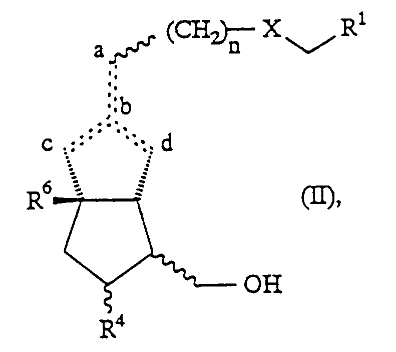 dans laquelle a-b, b-c, b-d, R⁶, n et X ont les significations données ci-dessus et R¹ représente un groupe ester -COOR²- avec R² ayant la signification donnée ci-dessus à l'exception de l'hydrogène, pour obtenir un aldéhyde de formule III : 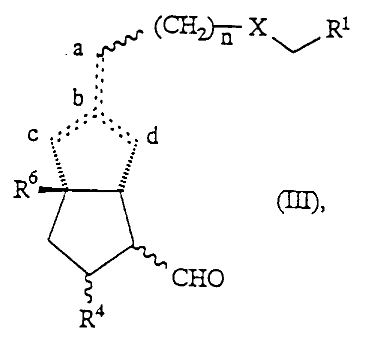 et on transforme avec un composé de formule IV : 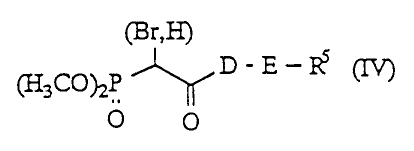 dans laquelle D, E et R⁵ ont les significations données ci-dessus, en un composé de formule V, dans laquelle a-b, b-c, b-d, R⁶, R⁴, D, E, R⁵, n et X ont les significations données ci-dessus et R¹ représente un groupe ester -COOR²- avec R² ayant la signification donnée ci-dessus à l'exception de l'hydrogène, on réduit la cétone formée et éventuellement on élimine le bromure d'hydrogène, on saponifie l'ester obtenu, on transforme en leurs sels avec des bases physiologiquement tolérables, on convertit avec de l'α, la β ou la γ-cyclodextrine en un clathrate ou on encapsule dans des liposomes.
